(19)

Europäisches
Patentamt
European
Patent Office
Office européen
des brevets

(11) **EP 2 241 619 A1**

(12) **EUROPEAN PATENT APPLICATION**

(43) Date of publication:
20.10.2010 Bulletin 2010/42

(51) Int Cl.:
*C12N 9/16* (2006.01)     *C12Q 1/48* (2006.01)

(21) Application number: 09005492.5

(22) Date of filing: 17.04.2009

(84) Designated Contracting States:
**AT BE BG CH CY CZ DE DK EE ES FI FR GB GR
HR HU IE IS IT LI LT LU LV MC MK MT NL NO PL
PT RO SE SI SK TR**
Designated Extension States:
**AL BA RS**

(71) Applicant: **Max-Planck-Gesellschaft zur
Förderung der
Wissenschaften e.V.
80539 München (DE)**

(72) Inventors:
• **Rauh, Daniel
  67549 Worms (DE)**
• **Simard, Jeffrey Raymond
  Salem, MA 01970 (US)**

(74) Representative: **Vossius & Partner
Siebertstrasse 4
81675 München (DE)**

(54) **Development of fluorescently P-loop labeled kinases for screening of inhibitors**

(57)     The present invention relates to a kinase labeled at an amino acid naturally present or introduced in the P-loop of said kinase, wherein said labeling is effected at a free thiol or amino group of said amino acid and said label is (a) a thiol- or amino-reactive fluorophore sensitive to polarity changes in its environment; or (b) a thiol-reactive spin label, an isotope or an isotope-enriched thiol- or amino-reactive label, such that said fluorophore, spin label, isotope or isotope-enriched label does not inhibit the catalytic activity and does not interfere with the stability of the kinase. The invention furthermore relates to a method of screening for kinase inhibitors, a method of determining the kinetics of ligand binding and/or of dissociation of a kinase inhibitor and a method of generating mutated kinases suitable for the screening of kinase inhibitors using the kinase of the present invention.

EP 2 241 619 A1

**Description**

The present invention relates to a kinase labeled at an amino acid naturally present or introduced in the P-loop of said kinase, wherein said labeling is effected at a free thiol or amino group of said amino acid and said label is (a) a thiol- or amino-reactive fluorophore sensitive to polarity changes in its environment; or (b) a thiol-reactive spin label, an isotope or an isotope-enriched thiol- or amino-reactive label, such that said fluorophore, spin label, isotope or isotope-enriched label does not inhibit the catalytic activity and does not interfere with the stability of the kinase. The invention furthermore relates to a method of screening for kinase inhibitors, a method of determining the kinetics of ligand binding and/or dissociation of a kinase inhibitor and a method of generating mutated kinases suitable for screening of kinase inhibitors using the labeled kinase of the present invention.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Protein kinases are an important set of enzymes regulating key cellular processes. The improved understanding of aberrantly regulated kinase signaling in cancer biology (Gschwind and Fischer, 2004) has lead to the development of small organic molecules that are used to specifically target unwanted kinase activities and initiated the area of targeted cancer therapies (Zhang et al., 2009). Most kinase inhibitors are Type I inhibitors such as dasatinib (Sprycel®), bind to the active "DFG-in" conformation of the kinase and compete with ATP in order to form critical hydrogen bonds with the kinase hinge region. However, in addition to an already exhausted chemical space within the ATP binding site, poor inhibitor selectivity and efficacy as well as the emergence of drug resistance are bottlenecks in the development of effective long-term therapies. Current medicinal chemistry endeavors therefore seek to overcome these limitations by identifying new chemical entities that bind to allosteric sites and stabilize enzymatically incompetent kinase conformations. One of these sites is only present in the inactive "DFG-out" kinase conformation and is moving to the forefront of kinase research. The DFG-out conformation results from structural changes in the activation loop induced by an 180° flip of the highly-conserved DFG motif (Liu and Gray, 2006; Pargellis et al., 2002). Type II and Type III inhibitors bind to this less conserved allosteric site and are believed to have superior selectivity profiles, improved pharmacological properties (Copeland et al., 2006) and offer new opportunities for drug development (Liu and Gray, 2006). More specifically, Type II inhibitors such as sorafenib (Nexavar®, Wan et al., 2004), imatinib (Gleevec®, Nagar et al., 2002) and BIRB-796, a selective inhibitor of p38α (Pargellis et al., 2002), bind to the hinge region and are ATP-competitive but extend into this allosteric site while Type III inhibitors bind exclusively within the allosteric pocket (Pargellis et al., 2002; Simard et al., submitted). Until recently, approaches that allowed for the unambiguous identification of inhibitors which stabilize the inactive DFG-out conformation fell short or were not compatible with the high-throughput screening formats used by academia and industry to identify new hit compounds (Annis et al., 2004; Vogtherr et al., 2006).

The attachment of fluorophores to proteins is a well-established approach used to detect conformational changes in protein structure in response to ligand binding. In addition to the commercially-available probe *acrylodan-labeled fatty acid binding protein* (ADIFAB; Molecular Probes), which measures the concentration of unbound fatty acids in buffer (Richieri et al., 1999), this approach has been applied to various other proteins including acetylcholine binding protein (Hibbs et al., 2004), interleukin-1β (Yem et al., 1992) and various sugar and amino acid binding proteins (de Lorimier et al., 2002). It emanates from the above discussion that it would be desirable to have versatile means and methods for screening for specific kinase inhibitors. The solution to this technical problem is achieved by providing the embodiments characterized in the claims.

Accordingly, the present invention relates to a kinase labeled at an amino acid naturally present or introduced in the P-loop of said kinase, wherein said labeling is effected at a free thiol or amino group of said amino acid and said label is (a) a thiol- or amino-reactive fluorophore sensitive to polarity changes in its environment; or (b) a thiol-reactive spin label, an isotope or an isotope-enriched thiol- or amino-reactive label, such that said fluorophore, spin label, isotope or isotope-enriched label does not inhibit the catalytic activity and does not interfere with the stability of the kinase.

The term "kinase" is well-known in the art and refers to a type of enzyme that transfers phosphate groups from high-energy donor molecules, such as ATP, to specific target molecules such as proteins. Kinases are classified under the enzyme commission (EC) number 2.7. According to the specificity, protein kinases can be subdivided into serine/threonine kinases (EC 2.7.11, e.g. p38α), tyrosine kinases (EC 2.7.10, e.g. the EGFR kinase domain), histidine kinases (EC 2.7.13), aspartic acid/glutamic acid kinases and mixed kinases (EC 2.7.12) which have more than one specificity (e.g. MEK being specific for serine/threonine and tyrosine).

Amino acids are defined as organic molecules that have a carboxylic and amino functional group. They are the essential building blocks of proteins. Examples of amino acids having a free thiol group are cysteine, belonging to the 20 proteinogenic amino acids, and acetyl-cysteine being a non-standard amino acid rarely occurring in natural amino acid sequences. Proteinogenic amino acids having a free amino group are lysine, histidine or arginine and amino acids being aromatic amines, such as tryptophan. Pyrrolysine, 5-hydroxylysine or o-aminotyrosine are non-standard amino acids having a free amino group. The amino acids asparagine and glutamine, although having a free amino group, are not suitable in the present invention as they are not reactive to labeling agents and are thus excluded.

Tryptophan is an aromatic amino acid having an amino group in its indole ring. Aromatic amines are weak bases and thus unprotonated at pH 7. However, they can still be modified using a highly reactive reagent such as an isothiocyanate, sulfonyl chloride or acid

The kinase is labeled at a free thiol or amino group of an amino acid at the desired position in the kinase, i. e. in the P-loop. During labeling, the previously free thiol or amino group is involved in forming the covalent bond between the labeled amino acid and the label according to items (a) and (b).

Said amino acid to be labeled is located in the P-loop of the kinase. This means that only kinases having a P-loop or a structure equivalent thereto fall within the present invention. The P-loop (also called glycine-rich loop) is a highly flexible structural feature conserved among all ATP/GTP binding proteins (Saraste et al., 1990). In kinases, the P-loop contains the canonical Gly-X-Gly-X-X-Gly motif (where X is any amino acid) and is located in the N-terminal lobe of kinases where it serves as regulatory loop to guide the entry of ligands such as ATP into the ATP binding site of kinases (Wong et al., 2005).

Cysteines which are naturally present in a kinase of interest and are solvent-exposed can be located outside the P-loop or within the P-loop sequence. This equally applies to amino acids having a free amino group.

The modified kinase of the invention is labeled at an amino acid naturally present or introduced into the P-loop. If no suitable amino acid, i.e. one having a free thiol- or amino group, is present in the P-loop, said amino acid can be introduced, i.e. inserted by adding it or by replacing an existing amino acid, by techniques well-known in the art. In any case, it is to be understood for the avoidance of doubt that the amino acid is only labeled after its introduction into the P-loop if it is to be labeled by reaction with labeling reagents. The above techniques comprise site-directed mutagenesis as well as other recombinant, synthetic or semi-synthetic techniques. In case a non-standard amino acid is to be introduced into the kinase, an amino acid stretch containing said amino acid may be chemically synthesized and then connected to the remaining part(s) of the kinase which may have been produced recombinantly or synthetically.

The process of labeling involves incubation of the kinase, e.g. the mutated kinase of the invention (e.g. the kinase with a cysteine introduced in the P-loop), with a thiol- or amino-reactive label under mild conditions resulting in the labeling of said mutated kinase at the desired position in the P-loop. Mild conditions refer to buffer pH (e.g. around pH7 for thiol-reactive probes), ratio of label to kinase, temperature and length of the incubation step (for thiol-reactive probes e.g. 4 °C and overnight in the dark) which are known to the skilled person and provided with instruction manuals of providers of thiol- and amino-reactive probes. Such conditions need to be optimized to slow down the reaction of the chosen thiol- or amino-reactive label to ensure that labeling of said kinase is specific to the desired labeling site. In the case of fluorophore labeling, it is necessary to carry out the incubation in the dark. Increased light exposure results in bleaching of the fluorophore and a less intense fluorescence emission. After labeling, the labeled kinase is preferably concentrated, purified by gel filtration experiments or washed several times with buffer to remove excess unreacted label. The wash buffer is typically the buffer used to store the labeled kinase and may also be the buffer in which the desired measurements are made.

The term "fluorophore" denotes a molecule or functional group within a molecule which absorbs energy such as a photon of a specific wavelength and emits energy, i.e. light at a different (but equally specific) wavelength (fluorescence) immediately upon absorbance (unlike the case in phosphorescence) without the involvement of a chemical reaction (as the case in bioluminescence). Usually the wavelength of the absorbed photon is in the ultraviolet range but can reach also into the infrared range. The wavelength of the emitted light is usually in the visible range. The amount and wavelength of the emitted energy depend primarily on the properties of the fluorophore but may also be influenced by the chemical environment surrounding the fluorophore. A number of fluorophores are sensitive to changes in their environment. This includes changes in the polarity, charge and/or in the conformation of the molecule they are attached to. Fluorescence occurs when a molecule relaxes to its ground state after being electrically excited which, for commonly used fluorescent compounds that emit photons with energies from the UV to near infrared, happens in the range of between 0.5 and 20 nanoseconds.

The term "thiol- or amino-reactive" denotes the property of a compound, e.g. a fluorophore, to specifically react with free thiol- or amino groups. This is due to a functional group present in said compound which directs a specific reaction with a thiol or amino group. These functional groups may be coupled to molecules such as fluorophores, spin labels or isotope-enriched molecules in order to provide specific labels attachable to free thiol- or amino-groups. Examples for thiol-specific compounds are e.g. haloalkyl compounds such as iodoacetamide, maleimides, Hg-Link™ phenylmercury compounds or TS-link™ reagents (both Invitrogen). Haloalkyl compounds react with thiol or amino groups depending on the pH.

The term "spin label" (SL) denotes a molecule, generally an organic molecule, which possesses an unpaired electron, usually on a nitrogen atom, and has the ability to bind to another molecule. Spin labels are used as tools for probing proteins using EPR spectroscopy. The site-directed spin labeling (SDSL) technique allows one to monitor the conformation and dynamics of a protein. In such examinations, amino acid-specific SLs can be used.

Site-directed spin labeling is a technique for investigating protein local dynamics using electron spin resonance. SDSL is based on the specific reaction of spin labels with amino acids. A spin label built in protein structures can be detected by EPR spectroscopy. In SDSL, sites for attachment of spin labels such as thiol or amino groups, if not naturally present, are introduced into recombinantly expressed proteins by site-directed mutagenesis. Functional groups contained within the spin label determine their specificity. At neutral pH, protein thiol groups specifically react with functional groups such as methanethiosulfonate, maleimide and iodoacetamide, creating a covalent bond with the amino acid cysteine. Spin labels are unique molecular reporters, in that they are paramagnetic, i.e. they contain an unpaired electron. Nitroxide spin labels are widely used for the study of macromolecular structure and dynamics because of their stability and simple EPR signal. The nitroxyl radical (N-O) is usually incorporated into a heterocyclic ring such as pyrrolidine, and the unpaired electron is predominantly localized to the N-O bond. Once incorporated into the protein, a spin label's motions are dictated by its local environment. Because spin labels are exquisitely sensitive to motion, this has profound effects on the EPR spectrum of the spin-label attached to the protein.

The signal arising from an unpaired electron can provide information about the motion, distance, and orientation of unpaired electrons in the sample with respect to each other and to the external magnetic field. For molecules free to move in solution, EPR works on a much faster time-scale than NMR (Nuclear Magnetic Resonance spectroscopy), and so can reveal details of much faster molecular motions, i.e. nanoseconds as opposed to microseconds for NMR. The gyromagnetic ratio of the electron is orders of magnitude larger than of nuclei commonly used in NMR, and so the technique is more sensitive, though it does require spin labeling.

The term "isotope" denotes a chemical species of a chemical element having different atomic mass (mass number) than the most abundant species of said element. Isotopes of an element have nuclei with the same number of protons (the same atomic number) but different numbers of neutrons.

Isotopes suitable for EPR or NMR need to have a nonzero nuclear spin. The most common isotopes currently used are $^{1}H$, $^{2}D$, $^{15}N$, $^{13}C$, and $^{31}P$.

The term "isotope-enriched" denotes that a compound, e.g. a thiol- or amino-reactive label has been synthesized using or reacted with an isotope so that said isotope is introduced into said compound. The compound may comprise one or more isotopes of one or more different species.

The label has to be positioned so that it does not inhibit the kinase's catalytic activity and does not interfere with its stability. In the case of a kinase that is isotopically labeled on an amino acid, e.g. a cysteine, and produced by growing host organisms expressing the kinase with isotopically labeled amino acid already incorporated into the sequence, inhibition of the activity or interference with the stability of the kinase is unlikely. On the other hand, care also has to be taken when selecting the position in the P-loop where the label is to be introduced. If no suitable amino acid is present at the position of choice, the amino acid present at said position must be replaced with an amino acid containing a free thiol or amino group other than the $\alpha$-amino group involved in peptide bonds. The P-loop confers ATPase activity to the kinase. Accordingly, a suitable labeling position should be chosen such that the kinase retains at least 70%, preferably at least 80%, more preferably at least 90%, and most preferably 100% of its ATPase activity. Tests of how to evaluate the activity and stability of a kinase prior to and after replacement of an amino acid are well known to the skilled person and include visual inspection of the purified protein, circular dichroism (CD) spectroscopy, crystallization and structure determination, enzyme activity assays, protein melting curves, differential scanning calorimetry and NMR spectroscopy.

As described above, the P-loop comprises a highly conserved glycine-rich motif G-X-G-X-X-G, also called the ATP/GTP phosphate binding motif in ATPases/GTPases, respectively. The conserved glycines are suggested to be critical for optimal positioning of the phosphates of ATP or GTP for efficient phospho transfer to the docked substrate of the enzyme. Although, in principle, any amino acid within said conserved motif could be chosen for replacement and/or labeling according to the invention, it is preferred that a less-conserved amino acid at the variable positions in the glycine-rich motif (designated as x) is chosen. Choosing one of the conserved glycine residues might interfere with the ATPase activity of the kinase which should preferably be avoided in order to obtain a labeled kinase with at least similar, preferably essentially unaltered catalytic activity as compared to the naturally occurring kinase as also described above. It is further preferred that the amino acid at a position X to be replaced is an aromatic amino acid such as phenylalanine or a tyrosine. Both phenylalanine and tyrosine are bulky and are expected to adopt similar conformational rearrangements with ligand binding when compared to the covalently attached labels according to the invention, in particular the thiol-reactive label acrylodan.

In this regard, no inhibition of the catalytic activity is present if at least 90% of the catalytic activity of the kinase are retained, preferably at least 95%, more preferably at least 98%. Most preferably, the catalytic activity of the kinase is fully retained. The term "does not inhibit the catalytic activity" is thus, in some embodiments where the catalytic activity amounts to less than 100 %, to be equated with and having the meaning of "does not essentially interfere with the catalytic activity". Regarding stability, the amino acid introduced does not interfere with the essential intramolecular contacts that ensure structural stability of the protein, so that the kinase can carry out the biological function described herein.

To overcome the drawbacks of presently existing screening methods, the present invention involves a labeling strategy to create e.g. fluorescent-tagged kinases which (i) are highly sensitive to the binding of kinase inhibitors, (ii) can be used to measure the kinetics of ligand binding and dissociation in real-time, (iii) can be used to directly measure the Kd of these ligands and (iv) is rapid, robust, reproducible and adaptable to high-throughput screening methods.

In contrast to the prior art and as demonstrated in the appended examples, the present invention provides kinases and screening methods using these kinases which enables for screening for inhibitors with a reduced effort and material and as well as a superior reliability. This is essentially achieved by providing a labeling strategy for a kinase such that the label alters its behavior in reaction to changes in its environment caused e.g. by conformational changes in the P-loop of the kinase.

Besides conventional kinase assays for the screening of modulators of kinase activity, various approaches have recently been developed. However, many of these approaches suffer from major drawbacks. For example, Annis et al. (2004) describe an approach using affinity selection-mass spectrometry (AS-MS). This method is described as suitable for high-throughput screening. However, a size exclusion chromatography step has to be applied prior to the examination of each probe which is time-consuming and requires a lot of material.

De Lorimier et al. (2002) describe a family of biosensors based on bacterial proteins binding to small molecule ligands which were modified and labeled with different environmentally sensitive fluorophores. Upon ligand binding, the fluorophores alter their emission wavelength and/or intensity thus indicating the presence and/or concentration of the specific ligand bound to a probe. However, the labeling of kinases and the use of said kinases in the screening for specific inhibitors is neither disclosed nor suggested.

The principle underlying the present invention is that the P-loop reacts to conformational changes of the activation loop upon binding of a type II or type III inhibitor. The activation loop is a flexible segment near the entrance to the active site which forms the substrate binding cleft of most kinases and can be phosphorylated on one or more amino acids to provide an important regulatory mechanism throughout the protein kinase superfamily (Johnson and Lewis, 2001; Taylor and Radzio-Andzelm, 1994; Johnson et al., 1996). The activation loop consists of several amino acids which form a loop that is flexible in most kinases which begins with a highly-conserved aspartate-phenylalanine-glycine (DFG) motif in the ATP binding site and extends out between the N- and C-lobes of the kinase. The activation loop is a structural component crucial for enzymatic kinase activity. It is part of the substrate binding cleft and contains several amino acid residues which assist in the recognition of specific substrates and also contains serines, threonines or tyrosines which can be phosphorylated. The conformation of the activation loop is believed to be in dynamic equilibrium between the DFG-in (active kinase) and DFG-out (inactive kinase) conformations. Phosphorylation and/or binding of interaction partners (other proteins or DNA) result in a shift of the equilibrium. In the DFG-in conformation, the aspartate contained in the motif is pointed into the ATP binding site and the adjacent phenylalanine is pointed away from the ATP site and into the an adjacent allosteric site. When the conserved DFG motif forming part of the activation loop adopts the inconformation, ATP-competitive inhibitors (Type I inhibitors) can bind to the kinase. In the DFG-out conformation, the positions of these residues are flipped 180˚ in orientation. The out-conformation of the activation loop prevents ATP and substrate binding.

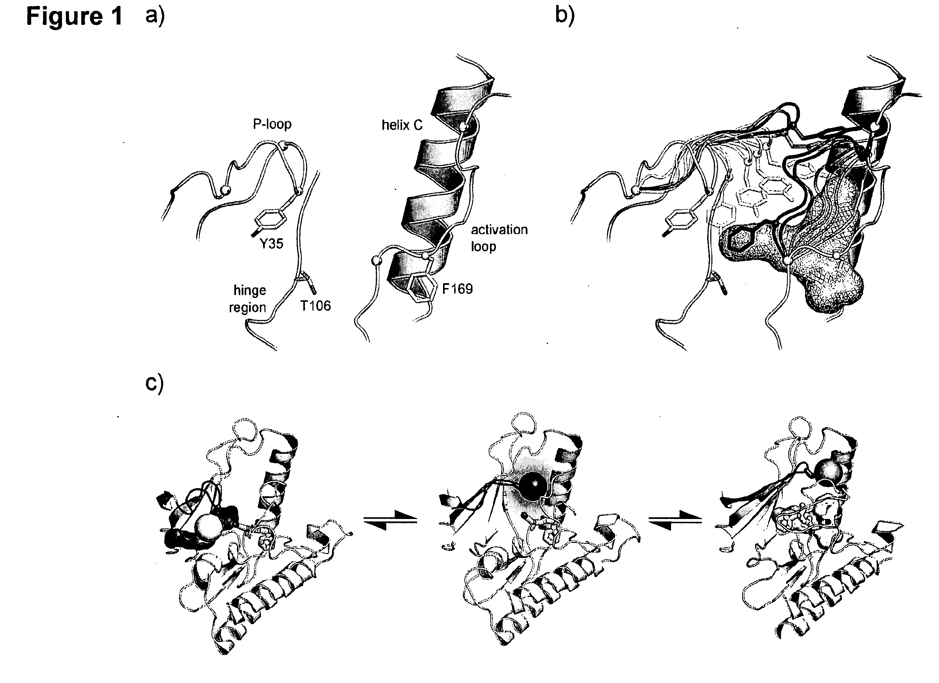

Besides controlling the entry of ligands and substrates into the ATP binding sites as described above, the P-loop helps to shield ATP and other ligands from the surrounding solvent. It has been shown to adopt various conformations related to the binding of some Type I inhibitors in the ATP binding pocket (Hanks and Hunter, 1995 (Hanks and Hunter, 1995; Mapelli et al., 2005). in accordance with the present invention allosteric inhibitors ligands (see Figure 1c) were detected. Further, a fluorescent- or spin-labeled P-loop assay system is provided. The attached fluorophore or spin label reports movements in the P-loop which occur when the activation loop of the kinase adopts the DFG-out conformation. As shown in the appended example, the introduction of a Cys residue *via* site-directed mutagenesis into the position directly preceding the third Gly of the Gly-X-Gly-X-X-Gly motif to specifically label the P-loop with the environmentally-sensitive fluorophore acrylodan results in a kinase having the ability to aid in screening for inhibitors. The residue at this site is conserved as a Tyr or Phe in approximately 80% of all human kinases, suggesting a role for the aromatic ring system of these side chains in mediating the cross-talk of this loop with other structural features and ligands. More importantly, this observation suggested that introduction of the planar ring system of acrylodan would be well tolerated by the kinase.

The present inventors recently developed a robust assay system in which they tagged the activation loop of target kinases ( co-pending applications EP 08 01 3340 and EP 08 02 0341), allowing for the first time Type III ligands of cSrc and p38α to be identified and led to the development of potent Type II inhibitors of gatekeeper mutated drug resistant cSrc-T338M. These earlier studies highlight the far-reaching implications of assays which can be used to screen for and enrich these types of ligands. However, in order to avoid potential changes in kinase activity resulting from alterations in the DFG-in/out conformational equilibrium upon labeling of the activation loop, the alternative labeling strategy for identifying and characterizing Type II and Type III inhibitors as provided by the present invention makes said changes in activity less likely.

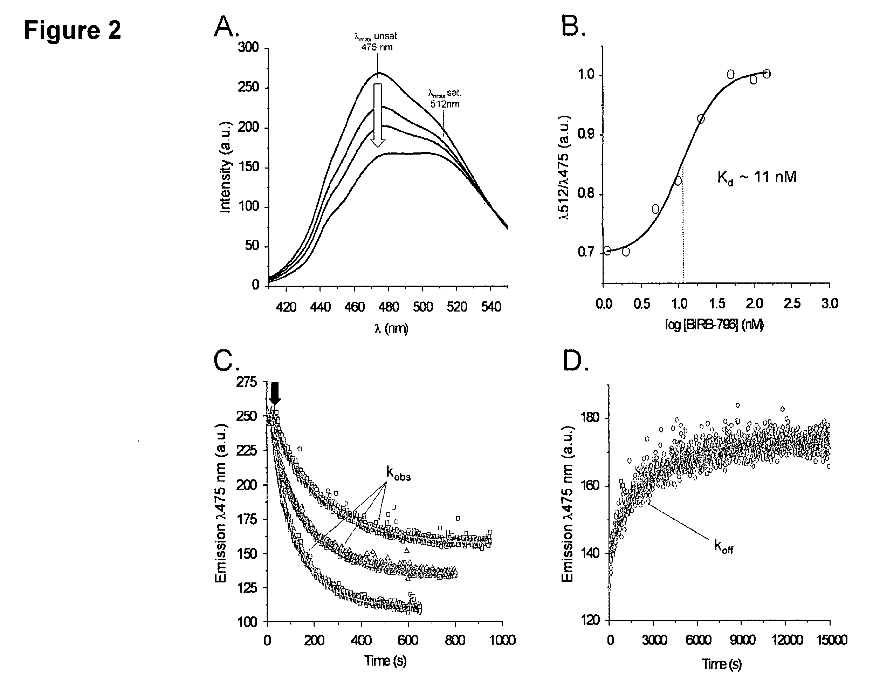

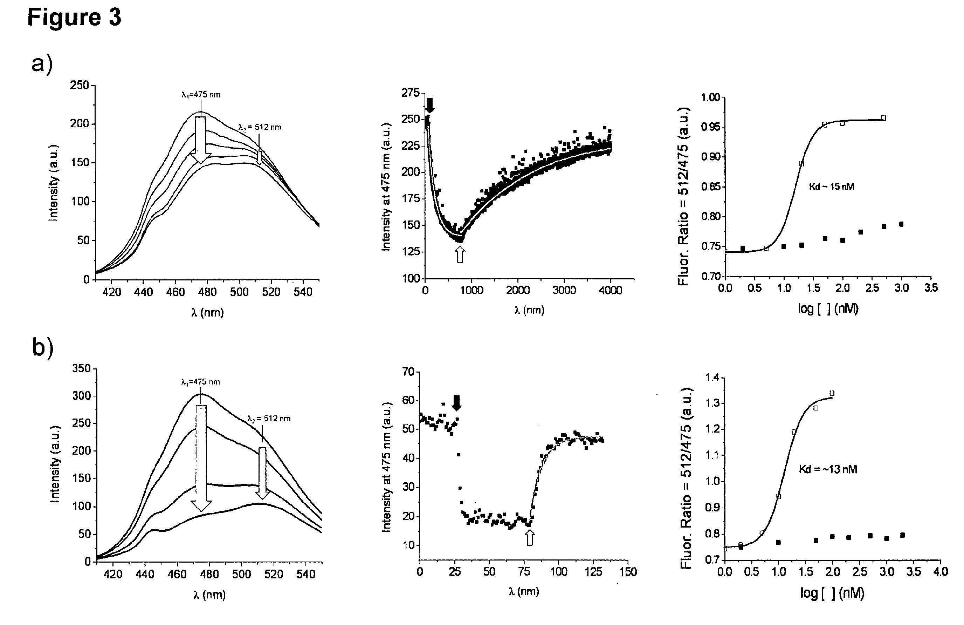

As shown in the appended examples, the present invention demonstrates the ability of P-loop labeled kinases to sensitively detect Type II and Type III inhibitors which induced changes in the environment of the fluorophore and altered its fluorescence properties (see Figure 2a). The present assay is also able to strongly detect Type I ligands which stabilize the DFG-out conformation by way of a unique binding mode. Such ligands bind within the ATP-binding site but utilize a unique ring-stacking interaction which forms between the inhibitor molecule, the highly-conserved Phe of the DFG motif and the planar ring system of the residue typically found at the chosen labeling position in the P-loop (Tyr35 in p38$\alpha$). Lastly, some Type I inhibitors which bind to the DFG-in conformation have been shown to directly interact with the described Tyr/Phe side chain of the P-loop (Tamayo et al. 2005). By using this position to label the kinase, the detection of these types of inhibitors is also possible (Figure 3B right panel), without inducing the DFG-out conformation or movement of the activation loop. In comparison to the recently reported assay in which the activation loop is directly labeled with a fluorophore (patent applications EP 08 01 3340 and EP 08 02 0341), this assay system provides a powerful alternative screening tool for detecting changes in the activation loop conformation correlated with ligand binding.

In a preferred embodiment, the kinase is a serine/threonine kinase or a tyrosine kinase.

In another preferred embodiment, the kinase is MEK kinase, CSK, an Aurora kinase, GSK-3$\beta$, cSrc, EGFR, Abl, DDR1, LCK, a CDK, p38$\alpha$ or another MAPK.

Mitogen-activated protein (MAP) kinases (EC 2.7.11.24) are serine/threonine-specific protein kinases that respond to extracellular stimuli (mitogens) and regulate various cellular activities, such as gene expression, mitosis, differentiation, and cell survival/apoptosis. Extracellular stimuli lead to activation of a MAP kinase via a signaling cascade ("MAPK cascade") composed of a MAP kinase, MAP kinase kinase (MKK or MAP2K) and MAP kinase kinase kinase (MKKK or MAP3K, EC 2.7.11.25).

A MAP3K that is activated by extracellular stimuli phosphorylates a MAP2K on its serine and/or threonine residues, and then this MAP2K activates a MAP kinase through phosphorylation on its serine and/or tyrosine residues. This MAP kinase signaling cascade has been evolutionarily well-conserved from yeast to mammals.

To date, six distinct groups of MAPKs have been characterized in mammals:

1. extracellular signal-regulated kinases (ERK1, ERK2). The ERK (also known as classical MAP kinases) signaling pathway is preferentially activated in response to growth factors and phorbol ester (a tumor promoter), and regulates cell proliferation and cell differentiation.
2. c-Jun N-terminal kinases (JNKs), (MAPK8, MAPK9, MAPK10), also known as stress-activated protein kinases (SAPKs).
3. p38 isoforms are p38$\alpha$ (MAPK14), p38$\beta$ (MAPK11), p38$\gamma$ (MAPK12 or ERK6) and p38$\delta$ (MAPK13 or SAPK4). Both JNK and p38 signaling pathways are responsive to stress stimuli, such as cytokines, ultraviolet irradiation, heat shock, and osmotic shock, and are involved in cell differentiation and apoptosis. p38$\alpha$ MAP Kinase (MAPK), also called RK or CSBP, is the mammalian orthologue of the yeast HOG kinase which participates in a signaling cascade controlling cellular responses to cytokines and stress. Similar to the SAPK/JNK pathway, p38 MAP kinase is activated by a variety of cellular stresses including osmotic shock, inflammatory cytokines, lipopolysaccharides (LPS), ultraviolet light and growth factors. p38 MAP kinase is activated by phosphorylation at Thr180 and Tyr182.
4. ERK5 (MAPK7), which has been found recently, is activated both by growth factors and by stress stimuli, and it participates in cell proliferation.
5. ERK3 (MAPK6) and ERK4 (MAPK4) are structurally related atypical MAPKs which possess an SEG (serine - glutamic acid - glycine) motif in the activation loop and display major differences only in the C-terminal extension.
6. ERK7/8 (MAPK15) are the most recently discovered members of the MAPK family and behave similar to ERK3/4.

Mitogen-activated protein kinase kinase forms a family of kinases which phosphorylates mitogen-activated protein kinase. They are also known as MAP2K and classified as EC 2.7.12.2. Seven genes exist. These encode MAP2K1 (MEK1), MAP2K2 (MEK2), MAP2K3 (MKK3), MAP2K4 (MKK4), MAP2K5 (MKK5), MAP2K6 (aka MKK6), MAP2K7 (MKK7). The activators of p38 (MKK3 and MKK4), JNK (MKK4), and ERK (MEK1 and MEK2) define independent MAP kinase signal transduction pathways.

Aurora kinases A (also known as Aurora, Aurora-2, AIK, AIR-1, AIRK1, AYK1, BTAK, Eg2, MmIAK1, ARK1 and STK15), B (also known as Aurora-1, AIM-1, AIK2, AIR-2, AIRK-2, ARK2, IAL-1 and STK12) and C (also known as AIK3) participate in several biological processes, including cytokinesis and dysregulated chromosome segregation. These important regulators of mitosis are over-expressed in diverse solid tumors. One member of this family of serine/threonine kinases, human Aurora A, has been proposed as a drug target in pancreatic cancer. The recent determination of the three-dimensional structure of Aurora A has shown that Aurora kinases exhibit unique conformations around the activation loop region. This property has boosted the search and development of inhibitors of Aurora kinases, which might also function as novel anti-oncogenic agents.

Glycogen synthase kinase 3 (GSK-3) is a serine/threonine protein kinase which in addition to the serine/threonine kinase activity has the unique ability to auto-phosphorylate on tyrosine residues. The phosphorylation of target proteins by GSK-3 usually inhibits their activity (as in the case of glycogen synthase and NFAT). GSK-3 is unusual among the kinases in that it usually requires a "priming kinase" to first phosphorylate a target protein and only then can GSK-3 additionally phosphorylate the target protein. In mammals GSK-3 is encoded by two known genes, GSK-3 alpha and beta. Aside from roles in pattern formation and cell proliferation during embryonic development, there is recent evidence for a role in tumor formation via regulation of cell division and apoptosis. Human glycogen synthase kinase-3 beta (GSK3β) is also associated with several pathophysiological conditions such as obesity, diabetes, Alzheimer's disease and bipolar disorder.

The Src family of proto-oncogenic tyrosine kinases transmit integrin-dependent signals central to cell movement and proliferation. The Src family includes nine members: Src, Lck, Hck, Fyn, Blk, Lyn, Fgr, Yes, and Yrk. These kinases have been instrumental to the modern understanding of cancer as a disease with disregulated cell growth and division. The cSrc proto-oncogene codes for the cSrc tyrosine kinase. Besides its kinase domain, cSrc is further comprised of an SH2 domain and an SH3 domain, which act as adaptor proteins for the formation of multi-enzyme complexes with the Src kinase domain. These domains are also involved in the auto-inhibition of the cSrc kinase domain. Mutations in this gene could be involved in the malignant progression of cancer cells. This protein specifically phosphorylates Tyr-504 residue on human leukocyte-specific protein tyrosine kinase (Lck), which acts as a negative regulatory site. It may also act on the Lyn and Fyn kinases.

Leukocyte-specific protein tyrosine kinase (Lck) is a protein that is found inside lymphocytes such as T-cells. Lck is a tyrosine kinase which phosphorylates tyrosine residues of certain proteins involved in the intracellular signaling pathways of lymphocytes. The N-terminal tail of Lck is myristoylated and palmitoylated, which tethers the protein to the plasma membrane of the cell. The protein furthermore contains an SH3 domain, an SH2 domain and in the C-terminal part the tyrosine kinase domain. The tyrosine phosphorylation cascade initiated by Lck culminates in the intracellular mobilization of calcium (Ca$^{2+}$) ions and activation of important signaling cascades within the lymphocyte. These include the Ras-MEK-ERK pathway, which goes on to activate certain transcription factors such as NFAT, NFκB, and AP-1 which then regulate the production of a plethora of gene products, most notably, cytokines such as Interleukin-2 that promote long-term proliferation and differentiation of the activated lymphocytes. Aberrant expression of Lck has been associated with thymic tumors, T-cell leukemia and colon cancers.

The catalytic activity of the Src family of tyrosine kinases is suppressed by phosphorylation on a tyrosine residue located near the C terminus (Tyr 527 in cSrc), which is catalyzed by C-terminal Src Kinase (Csk). Given the promiscuity of most tyrosine kinases, it is remarkable that the C-terminal tails of the Src family kinases are the only known targets of Csk. Interactions between Csk and cSrc, most likely representative for Src kinases, position the C-terminal tail of cSrc at the edge of the active site of Csk. Csk cannot phosphorylate substrates that lack this docking mechanism because the conventional substrate binding site used by most tyrosine kinases to recognize substrates is destabilized in Csk by a deletion in the activation loop (Levinson, 2008).

The epidermal growth factor receptor (EGFR; ErbB-1; HER1 in humans) is the cell-surface receptor for members of the epidermal growth factor family (EGF-family) of extracellular protein ligands. The epidermal growth factor receptor is a member of the ErbB family of receptors, a subfamily of four closely related receptor tyrosine kinases: EGFR (ErbB-1), HER2/c-neu (ErbB-2), Her 3 (ErbB-3) and Her 4 (ErbB-4). Active EGFR occurs as a dimer. EGFR dimerization is induced by ligand binding to the extracellular receptor domain and stimulates its intrinsic intracellular protein-tyrosine kinase activity. As a result, autophosphorylation of several tyrosine residues in the C-terminal (intracellular) domain of EGFR occurs. This autophosphorylation elicits downstream activation and signaling by several other proteins that associate with the phosphorylated tyrosines through their own phosphotyrosine-binding SH2 domains. The kinase domain of EGFR can also cross-phosphorylate tyrosine residues of other receptors it is aggregated with, and can itself be activated in that manner. The EGFR signaling cascade activates several downstream signaling proteins which then initiate several signal transduction cascades, principally the MAPK, Akt and JNK pathways, leading to DNA synthesis and cell proliferation. Such pathways modulate phenotypes such as cell migration, adhesion, and proliferation. Mutations that lead to EGFR overexpression (known as upregulation) or overactivity have been associated with a number of cancers. Consequently, mutations of EGFR have been identified in several types of cancer, and it is the target of an expanding class of anticancer therapies.

The ABL1-protooncogene encodes a cytoplasmic and nuclear protein tyrosine kinase that has been implicated in processes of cell differentiation, cell division, cell adhesion and stress response. The activity of c-Abl protein is negatively regulated by its SH3 domain. A genetic deletion of the SH3 domain turns ABL1 into an oncogene. This genetic deletion, caused by the (9;22) gene translocation results in the head-to-tail fusion of the BCR (MIM:151410) and ABL1 genes present in many cases of chronic myelogeneous leukemia. The DNA-binding activity of the ubiquitously expressed ABL1 tyrosine kinase is regulated by CDC2-mediated phosphorylation, suggesting a cell cycle function for ABL1.

Discoidin domain receptor family, member 1, also known as DDR1 or CD167ȧ (cluster of differentiation 167a), is a receptor tyrosine kinase (RTK) that is widely expressed in normal and transformed epithelial cells and is activated by various types of collagen. This protein belongs to a subfamily of tyrosine kinase receptors with a homology region similar to the *Dictyostelium discoideum* protein discoidin I in their extracellular domain. Its autophosphorylation is achieved by all collagens so far tested (type I to type VI). In situ studies and Northern-blot analysis showed that expression of this encoded protein is restricted to epithelial cells, particularly in the kidney, lung, gastrointestinal tract, and brain. In addition, this protein is significantly over-expressed in several human tumors from breast, ovarian, esophageal, and pediatric brain.

The kinases described above are preferred embodiments because all of them are involved in the development of diseases such as cancer for which at present not suitable cure is available or an improved treatment regimen is desired.

Using p38α, a kinase for which structural information was available, the present inventors demonstrated the applicability of the labeled kinase of the invention for screening purposes. Unexpectedly, the kinase could be prepared for labeling with a minimum of effort but also the labeled kinase exerted the desired properties, i.e. the introduced label proved suitable for the detection of conformational changes induced by binding of a specific inhibitor, in this case the known inhibitor BIRB-796 and several smaller BIRB-796 analogs.

In another preferred embodiment, the amino acid labeled is cysteine, lysine, arginine or histidine.

Cysteine has a free thiol group, whereas lysine, arginine or histidine each possess at least one free amino group.

In another preferred embodiment, one or more solvent-exposed cysteines present outside the P-loop are deleted or replaced.

If more than one amino acid having a free thiol or amino group is present in a kinase of interest prior to labeling, specific labeling of the amino acid in the P-loop may not be possible. Therefore, as discussed above, amino acids present in the kinase and having a free thiol or amino group should be deleted or replaced with another amino acid not having a free thiol or amino group if they are predicted or shown to be solvent-exposed. Cysteines which are naturally present in a kinase of interest and are solvent-exposed can be located outside the P-loop, in which case they should be deleted or replaced with another amino acid not having a free thiol group. This equally applies to amino acids having a free amino group which should then be replaced with an amino acid not having a reactive free amino group. In case that one or more amino acids having a free amino group is already present in the P-loop, amino acids having a free amino group and present in the P-loop in addition to the amino acid to be labeled, should be replaced or deleted, whichever of these mutations to the kinase does not inhibit its catalytic activity or interfere with its stability.

The term "solvent-exposed" refers to the position of an amino acid in the context of the three dimensional structure of the protein of which it is a part. Amino acids buried within the protein body are completely surrounded by other amino acids and thus do not have any contact with the solvent. In contrast, solvent-exposed amino acids are partially or fully exposed to the surrounding solvent and are thus accessible to chemicals potentially able to modify them. This applies e.g. to thiol- or amino-reactive labels used in the present invention which can react with solvent-exposed amino acids having a free thiol- or amino-group.

The term "delete" refers to excision of an amino acid without replacing it with another amino acid whereas the term "replace" refers to the substitution of an amino acid with another amino acid. If an amino acid is replaced with another amino acid or deleted, the amino acid to be replaced or to be deleted is preferably chosen such that the amino acid deleted or replaced does not result in a kinase with inhibited catalytic activity and does not interfere with the stability of the resulting kinase.

In a more preferred embodiment, the kinase is p38α and a cysteine to be labeled is introduced at position 35 of SEQ ID NO: 1 and preferably the cysteines at positions 119 and 162 of SEQ ID NO: 1 are replaced with another amino acid not having a free thiol group such as serine.

In general, amino acid replacements should be conservative. For cysteine, this means that it is preferably replaced with serine. In general, replacements of amino acids with different amino acids may be evaluated in view of whether they are conservative using the PAM250 Scoring matrix. The matrix is frequently used to score aligned peptide sequences to determine the similarity of those sequences (Pearson, 1990).

As described above, if not naturally present, an amino acid having a free thiol- or amino group has to be introduced into the P-loop of a kinase. In the case of p38α, structural studies were carried out using the available crystal structures for p38α in both the activated (DFG-in) and inactivated (DFG-out) state. P38α does not possess a cysteine in the P-loop. The above structural studies suggested that a replacement of tyrosine with a cysteine at position 35, which is located in the P-loop, would not significantly influence the catalytic activity or stability of the kinase.

From co-pending application EP 08 01 3340, it was known that two cysteines at positions 119 and 162 of SEQ ID NO: 1 are both solvent-exposed. To avoid potential interferences of the signals recorded for two additional cysteines not located in the P-loop, these two cysteines are preferably replaced with another amino acid, preferably with an amino acid similar in size and structure, such as serine.

If a kinase homologous to p38α is used, the position of the amino acid to be replaced with cysteine may correspond to position 35 in SEQ ID NO: 1. To determine which position in a kinase corresponds to position 35 in SEQ ID NO: 1, sequence alignments of SEQ ID NO: 1 with the used kinase can be effected, e.g. using publicly available programs such as CLUSTALW.

In another preferred embodiment, the thiol- or amino-reactive fluorophore is an environmentally sensitive di-substituted naphthalene compound of which one of the two substituents is a thiol- or amino-reactive moiety. The term "environmentally sensitive" denotes the sensitivity of the fluorophore to the conditions in its environment which is expressed in an alteration in its fluorescence emission at one or more wavelengths or in its complete emission spectrum. Conditions causing such alteration are e.g. changes in the polarity or conformational changes in the activation loop and, accordingly, in the P-loop.

The above types of fluorophores typically exhibit changes in both intensity and a shift in the emission wavelength depending on the polarity of the surrounding environment. Examples of this class of fluorophores include 6-acryloyl-2-dimethylaminonaphthalene (Acrylodan), 6-bromoacetyl-2-dimethylamino-naphthalenebadan (Badan), 2-(4'-(iodoacetamido)anilino)naphthalene-6-sulfonic acid, sodium salt (IAANS), 2-(4'-maleimidylanilino)naphthalene-6-sulfonic acid, sodium salt (MIANS), 5-((((2-iodoacetyl)amino)ethyl)amino)naphthalene-1-sulfonic acid (1,5-IAEDANS) and 5-dimethylaminonaphthalene-1-sulfonyl aziridine (dansyl aziridine) or a derivative thereof.

Other fluorophores which may be used due to their environmental sensitivity are coumarin-based compounds, benzoxadiazole-based compounds, dapoxyl-based compounds, biocytin-based compounds, fluorescein, sulfonated rhodamine-based compounds such as AlexaFluor dyes (Molecular Probes), Atto fluorophores (Atto Technology) or Lucifer Yellow. Coumarin-based fluorophores are moderately sensitive to environment and 7-diethylamino-3-(4'-maleimidylphenyl)-4-methylcoumarin (CPM) is an example. Benzoxadiazole fluorophores are also commonly used for forming protein-fluorophore conjugates and have a strong environmental dependence with 7-fluorobenz-2-oxa-1,3-diazole-4-sulfonamide (ABD-F) and N-((2-(iodoacetoxy)ethyl)-N-methyl)amino-7-nitrobenz-2-oxa-1,3-diazole ester (IANBD) as examples. PyMPO maleimide (for thiols) or succinimide ester (for amines) and various other dapoxyl dyes have good absorptivity and exceptionally high environmental sensitivity. Examples are 1-(2-maleimidylethyl)-4-(5-(4-methoxyphenyl)oxazol-2-yl)pyridinium methanesulfonate (PyMPO-maleimide), 1-(3-(succinimidyloxycarbonyl)benzyl)-4-(5-(4-methoxyphenyl) oxazol-2-yl) pyridinium bromide (PyMPO-succinimidyl ester) and Dapoxyl (2-bromoacetamidoethyl) sulphonamide. However, due to their longer, more flexible structures, these probes may effect P-loop movement or interactions between the P-loop and activation loop depending on the labeling site chosen. The applicability of the above substances depends on the individual kinase and the position of the amino acid to be labeled so that they can in principle be applied as labels as well, even if in some cases they may cause a reduced sensitivity in the methods of the invention. Matching the above substances with a suitable kinase can be performed by the skilled artisan using routine procedures in combination with the teachings of this invention.

In general, any fluorophore can be used as long as it does not inhibit the catalytic activity or interfere with the stability of the kinase. This means that the fluorophore is preferably not bulky or extended.

In a further preferred embodiment, the thiol-reactive spin-label is a nitroxide radical.

The dominant method for site-specifically labeling protein sequences with a spin-label is the reaction between methanethiosulfonate spin label and cysteine, to give the spin-labeled cysteine side chain, CYS-SL:

$$MeS(O)2SSR + R'SH ---> R'SSR + MeS(O)2SH$$

where R is the nitroxide group and R'SH is a protein with a cysteine sulfhydryl, and R'SSR is the spin-labeled protein. The cysteines for labeling are placed in the desired sequence position either through solid-phase techniques or through standard recombinant DNA techniques.

The present invention furthermore relates to a method of screening for kinase inhibitors comprising (a) providing a (fluorescently or spin-labeled or isotope-labeled) kinase according to the invention; (b) contacting said (fluorescently or spin-labeled or isotope-labeled) kinase with a candidate inhibitor; (c) recording the fluorescence emission signal at one or more wavelengths or a spectrum of said fluorescently labeled kinase of step (a) and step (b) upon excitation; or (c)' recording the electron paramagnetic resonance (EPR) or nuclear magnetic resonance (NMR) spectra of said spin-labeled or isotope-labeled kinase of step (a) and step (b); and (d) comparing the fluorescence emission signal at one or more wavelengths or the spectra recorded in step (c) or the EPR or NMR spectra recorded in step (c)'; wherein a difference in the fluorescence intensity at at least one wavelength, preferably at the emission maximum and/or a shift in the fluorescence emission wavelength in the spectra of said fluorescently labeled kinase obtained in step (c), or an alteration in the EPR or NMR spectra of said spin-labeled or isotope-labeled kinase obtained in step (c)' indicates that the candidate inhibitor is a kinase inhibitor.

Kinase inhibitors are substances capable of inhibiting the activity of kinases. They can more specifically inhibit the action of a single kinase, e.g. if they are allosteric inhibitors (Type III) or those binding to the allosteric site adjacent to the ATP-binding site and reaching into the ATP-binding pocket (Type II). Alternatively, an inhibitor can inhibit the action of a number of protein kinases, which is particularly the case if it binds exclusively to the ATP-binding pocket (Type I), which is very conserved among protein kinases.

A candidate inhibitor may belong to different classes of compounds such as small organic or inorganic molecules, proteins or peptides, nucleic acids such as DNA or RNA. Such compounds can be present in molecule libraries or designed from scratch. Small molecules according to the present invention comprise molecules with a molecular weight of up to 2000 Da, preferably up to 1500 Da, more preferably up to 1000 Da and most preferably up to 500 Da.

Recording the fluorescence emission signal at one or more wavelengths or a spectrum is usually accomplished using a fluorescence spectrometer or fluorimeter. Fluorescence spectroscopy or fluorimetry or spectrofluorimetry is a type of electromagnetic spectroscopy which analyzes fluorescence, or other emitted light, from a sample. It involves using a beam of light, usually ultraviolet light, that excites the electrons in certain molecules and causes them to emit light of a lower energy upon relaxation, typically, but not necessarily, visible light.

Two general types of instruments exist which can both be employed in the method of the invention: Filter fluorimeters use filters to isolate the incident light and fluorescent light, whereas spectrofluorimeters use diffraction grating monochromators to isolate the incident light and fluorescent light. Both types utilize the following scheme: The light from an excitation source passes through a filter or monochromator and strikes the sample. A proportion of the incident light is absorbed by the sample, and some of the molecules in the sample fluoresce. The fluorescent light is emitted in all directions. Some of this fluorescent light passes through a second filter or monochromator and reaches a detector, which is usually placed at 90° to the incident light beam to minimize the risk of transmitted or reflected incident light reaching the detector. Various light sources may be used as excitation sources, including lasers, photodiodes, and lamps; xenon and mercury vapor lamps in particular. The detector can either be single-channeled or multi-channeled. The single-channeled detector can only detect the intensity of one wavelength at a time, while the multi-channeled detects the intensity at all wavelengths simultaneously, making the emission monochromator or filter unnecessary. The different types of detectors have both advantages and disadvantages. The most versatile fluorimeters with dual monochromators and a continuous excitation light source can record both an excitation spectrum and a fluorescence spectrum. When measuring fluorescence spectra, the wavelength of the excitation light is kept constant, preferably at a wavelength of high absorption, and the emission monochromator scans the spectrum. For measuring excitation spectra, the wavelength passing though the emission filter or monochromator is kept constant and the excitation monochromator is scanning. The excitation spectrum generally is identical to the absorption spectrum as the fluorescence intensity is proportional to the absorption (for reviews see Rendell, 1987; Sharma and Schulman,1999; Gauglitz and Vo-Dinh, 2003; Lakowicz, 1999).

Nuclear magnetic resonance (NMR) is a physical phenomenon based upon the quantum mechanical magnetic properties of the nucleus of an atom. All nuclei that contain odd numbers of protons or neutrons have an intrinsic magnetic moment and angular momentum. The most commonly measured nuclei are hydrogen ($^1$H) (the most receptive isotope at natural abundance) and carbon ($^{13}$C), although nuclei from isotopes of many other elements (e.g. $^{113}$Cd, $^{15}$N, $^{14}$N $^{19}$F, $^{31}$P, $^{17}$O, $^{29}$Si, $^{10}$B, $^{11}$B, $^{23}$Na, $^{35}$Cl, $^{195}$Pt) can also be observed. NMR resonant frequencies for a particular substance are directly proportional to the strength of the applied magnetic field, in accordance with the equation for the Larmor precession frequency. NMR measures magnetic nuclei by aligning them with an applied constant magnetic field and perturbing this alignment using an alternating magnetic field, those fields being orthogonal. The resulting response to the perturbing magnetic field is the phenomenon that is exploited in NMR spectroscopy and magnetic resonance imaging, which use very powerful applied magnetic fields in order to achieve high spectral resolution, details of which are described by the chemical shift and the Zeeman Effect.

In the present invention, a suitable amino acid in the P-loop can be labeled with an isotope or thiol/amino-reactive small molecule containing enriched isotopes. In this case, the only signal comes from the enriched molecule on the P-loop, which is sensitive to protein conformation depending on the labeling site chosen.

Preferred isotopes are $^{13}$C, $^{15}$N, etc. which can be measured as 1D or 2D NMR spectra. Changes in protein conformation, e.g. due to the binding of an inhibitor will result in a shift of the NMR chemical shift(s) corresponding to the label.

Electron paramagnetic resonance (EPR) or electron spin resonance (ESR) spectroscopy, as has been briefly described above, is a technique for studying chemical species that have one or more unpaired electrons, such as organic and inorganic free radicals or inorganic complexes possessing a transition metal ion. The basic physical concepts of EPR are analogous to those of nuclear magnetic resonance (NMR), but it is electron spins that are excited instead of spins of atomic nuclei. Because most stable molecules have all their electrons paired, the EPR technique is less widely used than NMR. However, this limitation to paramagnetic species also means that the EPR technique is one of great specificity, since ordinary chemical solvents and matrices do not give rise to EPR spectra.

The EPR technique utilizes spin-labels. In this case, the kinase, to be examined is expressed in bacteria or other suitable host cells in the presence of an isotope such as $^{13}$C and $^{15}$N resulting in the incorporation of these isotopes throughout the entire protein as it is expressed. After purification of the isotope enriched protein, a spin label is attached to the P-loop as described above. In this case, 2D NMR spectra of the isotopes in the protein are recorded. As the P-loop and spin label change conformation, the spin label will induce a change in some of the protein signals coming from the incorporated isotopes which come into closer contact with the P-loop or spin label as inhibitors bind. Peaks would become broader as the spin label approaches.

Different EPR spectra or fluorescence emission signals at one or more wavelengths, preferably at the emission maximum, or different fluorescence emission spectra obtained in step (c) or (c)' indicate a conformational change in the kinase caused by binding of the candidate compound. This is due to the fact that binding of a compound to the allosteric site adjacent to the ATP-binding pocket, and in some cases to the ATP-binding pocket itself, results in a perturbation of the DFG motif, a conformational change in the activation loop and, accordingly, in the P-loop, a polarity change and/or a change in the interaction of free electrons in an attached spin-label with the nuclei of adjacent atoms. Upon comparison of the EPR or NMR spectra or the fluorescence emission, the present method reveals whether a candidate compound qualifies as a suitable kinase inhibitor, e.g. not only a high-affinity inhibitor but also one which specifically inhibits the activity of one kinase. The data recorded for the kinase without a candidate inhibitor and those recorded for the kinase having been contacted with said candidate inhibitor are compared. In case of fluorescence emission signal either the signal at one or more specific wavelengths can be recorded and compared enabling for a detection of a change in the intensity of the signal at the particular wavelength(s). Alternatively, a complete spectrum can be recorded and compared enabling also for the observation of changes in the maximum emission wavelength.

Preferably, said method is effected in high-throughput format. High-throughput assays, independently of being biochemical, cellular or other assays, generally may be performed in wells of microtiter plates, wherein each plate may contain 96, 384 or 1536 wells. Handling of the plates, including incubation at temperatures other than ambient temperature, and bringing into contact with test compounds, in this case putative inhibitors, with the assay mixture is preferably effected by one or more computer-controlled robotic systems including pipetting devices. In case large libraries of test compounds are to be screened and/or screening is to be effected within short time, mixtures of, for example 10, 20, 30, 40, 50 or 100 test compounds may be added to each well. In case a well exhibits inhibitory activity, said mixture of test inhibitors may be de-convoluted to identify the one or more test inhibitors in said mixture giving rise to said activity.

Alternatively, only one test inhibitor may be added to a well, wherein each test inhibitor is applied in different concentrations. For example, the test inhibitor may be tested in two, three or four wells in different concentrations. In this initial screening, the concentrations may cover a broad range, e.g. from 10 nM to 10 $\mu$M. The initial screening serves to find hits, i.e. test inhibitors exerting inhibiting activity at at least one concentration, preferably two, more preferably all concentrations applied, wherein the hit is more promising if the concentration at which an inhibitory activity can be detected is in the lower range. This alternative serves as one preferred embodiment in accordance with the invention.

Test inhibitors considered as a hit can then be further examined using an even wider range of inhibitor concentrations, e.g. 10 nM to 20 $\mu$M. The method applied for these measurements is described in the following.

The present invention furthermore relates to a method of determining the kinetics of ligand binding and/or of association or dissociation of a kinase inhibitor comprising (a) contacting a fluorescently labeled kinase according to the invention with different concentrations of an inhibitor; or (a)' contacting a fluorescently labeled kinase according to the invention bound to an inhibitor with different concentrations of unlabelled kinase; (b) recording the fluorescence emission signal at one or more wavelengths or a spectrum of said fluorescently labeled kinase for each concentration of inhibitor and/or unlabeled kinase upon excitation; (c) determining the rate constant for each concentration from the fluorescence emission signals at one or more wavelengths or the spectra recorded in step (b) or (c1) determining the $K_d$ from the fluorescence emission signal at one or more wavelengths or the spectra recorded in step (b) for each concentration of inhibitor; or (c2) determining the $K_a$ or inverse $K_d$ from the fluorescence emission signal at one or more wavelengths or the spectra recorded in step (b) for each concentration of unlabelled kinase; (d) directly determining the $k_{on}$ and/or extrapolating the $k_{off}$ from the rate constants determined in step (c) from the signals or spectra for the different concentrations of inhibitor obtained in step (b); or (d)' directly determining the $k_{off}$ and/or extrapolating the $k_{on}$ from the rate constants determined in step (c) from the signals or spectra for the different concentrations of unlabelled kinase obtained in step (b); and optionally (e) calculating the $K_d$ and/or $K_a$ from $k_{on}$ and $k_{off}$.obtained in step (d) or (d)'.

By contacting a labeled kinase with different concentrations of an inhibitor, and subsequently determining the fluorescence emission for each concentration applied, the binding affinity of an inhibitor can be measured. For each concentration, the ratio of bound and unbound inhibitor will be different, reflecting the increasing concentration of inhibitor but also the specific binding affinity of said inhibitor to said kinase.

The opposite approach can be followed by titrating a labeled kinase containing a bound inhibitor with unlabeled kinase with no inhibitor bound.

In chemical kinetics, a rate constant k quantifies the speed of a chemical reaction. For a chemical reaction where substance A and B are reacting to produce C, the reaction rate has the form:

$$\frac{d[C]}{dt} = k(T)[A]^m[B]^n$$

Wherein k(T) is the reaction rate constant that depends on temperature.

[A] and [B] are the concentrations of substances A and B, respectively, in moles per volume of solution assuming the reaction is taking place throughout the volume of the solution.

The exponents m and n are the orders and depend on the reaction mechanism. They can be determined experimentally.

A single-step reaction can also be described as:

$$\frac{d[C]}{dt} = A e^{\frac{-E_a}{RT}} [A]^m [B]^n$$

$E_a$ is the activation energy and R is the Gas constant. Since at temperature T the molecules have energies according to a Boltzmann distribution, one can expect the proportion of collisions with energy greater than $E_a$ to vary with $e^{-Ea/RT}$. $A$ is the pre-exponential factor or frequency factor.

$k_{on}$ and $k_{off}$ are constants that describe non-covalent equilibrium binding. When a ligand interacts with a receptor, or when a substrate interacts with an enzyme, the binding follows the law of mass action.

$$R + L \xrightarrow{k_{on}} \underset{k_{off}}{\longleftarrow} RL$$

In this equation R is the concentration of free receptor, L is the concentration of free ligand, and RL is the concentration of receptor-ligand complex. In the case of enzyme kinetics, R is the enzyme, or in this case a protein kinase, and L is the substrate, or in this case a candidate or known inhibitor. The association rate constant $k_{on}$ is expressed in units of $M^{-1}sec^{-1}$. The rate of RL formation equals R x L x $k_{on}$. The dissociation rate constant $k_{off}$ is expressed in units of $sec^{-1}$. The rate of RL dissociation equals RL x $k_{off}$. At equilibrium, the backward (dissociation) reaction equals the forward (association) reaction. Binding studies measure specific binding, which is a measure of RL. Enzyme kinetic assays assess enzyme velocity, which is proportional to RL, the concentration of enzyme-substrate complexes.

$$RL = R \cdot L \cdot \frac{k_{on}}{k_{off}}$$

The equilibrium dissociation constant, Kd is expressed in molar units and defined to equal $k_{off}/k_{on}$ to arrive at

$$RL = R \cdot L \cdot \frac{k_{on}}{k_{off}} = \frac{R \cdot L}{K_d}$$

The dissociation constant ($K_d$) corresponds to the concentration of ligand (L) at which the binding site on a particular protein is half occupied, i.e. the concentration of ligand, at which the concentration of protein with ligand bound (RL), equals the concentration of protein with no ligand bound (R). The smaller the dissociation constant, the more tightly bound the ligand is, or the higher the affinity between ligand and protein. Accordingly, the association constant $K_a$, also called inverse Kd, is defined as $1/k_d$. The dissociation constant for a particular ligand-protein interaction can change significantly with solution conditions (e.g. temperature, pH and salt concentration).

Depending on which sequence of steps is followed in the above method of the invention, the $K_d$ or $K_a$ can be measured directly or indirectly.

For directly measuring the $K_d$ or the $K_a$, respectively, step (c1) or (c2) which is the last step for this type of measurement follows step (b). This type of measurement is called endpoint measurement and also illustrated in the appended examples. Unlike for indirectly determining $K_d$ or $K_a$ through calculation using rate constants, the final fluorescence emission at equilibrium is measured rather than the fluorescence change over time. These measurements can be used to generate a binding curve using different inhibitor concentrations (for determining $K_d$) or concentrations of unlabelled kinase (for determining $K_a$). From these curves, $K_d$ or $K_a$ can be obtained directly. For indirectly obtaining $K_d$ or $K_a$, the rate constants from the fluorescence emission signal at one or more wavelengths or the spectra recorded in step (b) have to be determined for each concentration as done in step (c). Depending the type of titration, i.e. titration of labeled kinase with inhibitor or titration of labeled kinase bound to inhibitor with unlabeled kinase, either $k_{on}$ or $k_{off}$ can be determined directly from the measured rate constants. For determining $k_{on}$, step (d) is applied which also enables for extrapolation of $k_{off}$. Accordingly, step (d)' is applied for directly determining $k_{off}$ which in turn enables for extrapolation of $k_{on}$. From $k_{on}$ and/or $k_{off}$ obtained in steps (d) or (d)', the $K_d$ and/or $K_a$ can be calculated according to the equations discussed above.

The above method may also be applied in high-throughput screens. If a compound exerting inhibitory activity on a kinase has been identified, e.g. using the method of screening for kinase inhibitors of the invention, the present method can be used to further characterize said inhibitor. For example, the high-throughput format can be used to determine the Ka or Kd from the fluorescence emission signal at one or more wavelengths for multiple different concentrations of inhibitors (variant (a)) or, unlabelled kinase (variant (b)). Concentration ranges to be tested reach for example from 10 nM to 20 $\mu$M such that repeating series of 1, 2 and 5 (i.e. 10, 20, 50, 100, 200, 500 nM, etc.) between the concentrations assessed.

In a different embodiment, the present invention relates to a method of determining the dissociation or association of a kinase inhibitor comprising (a) contacting a spin-labeled or isotope-labeled kinase according to the invention with different concentrations of an inhibitor; or (a)' contacting a spin-labeled or isotope-labeled kinase according to the invention bound to an inhibitor with different concentrations of unlabelled kinase; (b) recording the EPR or NMR spectrum of said spin-labeled or isotope-labeled kinase for each concentration of inhibitor and/or unlabelled kinase; and (c) determining the $K_d$ from the EPR or NMR spectra recorded in step (b) for the different concentrations of inhibitor; or (c)' determining the $K_a$ from the EPR or NMR spectra recorded in step (b) for the different concentrations of unlabeled kinase.

Similar to the method disclosed further above relating to determining the kinetic constants using fluorescently labeled kinase, the present method allows for the direct determination of the association or dissociation constants for the reaction a kinase and an inhibitor. Unlike for fluorescently labeled kinases, the instrumental limitations and time required to collect NMR and EPR measurements are, in most cases, not compatible with the fast time scale of inhibitor binding and do not allow the direct determination of $k_{on}$ or $k_{off}$. Determinations for compounds which require several hours to bind to the kinase may also be possible.

The methods of the invention relating to determining kinetic data can also be applied to a high-throughput format. For example, a potential inhibitor identified with the screening method of the invention described above can be further characterized in that different concentrations of said inhibitor are applied to the kinase to determine the Kd. Suitable but not limiting concentration ranges for the inhibitor are between 10 nM and 20 $\mu$M.

More focused concentration ranges applied in the high-throughput format may serve to obtain more sensitive Kd measurements, e.g. with the cuvette approach and real-time kinetics measurements as done in the appended examples, by determining $k_{on}$ and $k_{off}$.

The present invention furthermore relates to a method of generating mutated kinases suitable for the screening of kinase inhibitors comprising (a) replacing solvent exposed amino acids having a free thiol or amino group, if any, present in a kinase of interest outside the P-loop or amino acids having a free thiol or amino group at an unsuitable position within the P-loop with an amino acid not having a free thiol or amino group; (b) mutating an amino acid in the P-loop of said kinase of interest to an amino acid having a free thiol or amino group if no amino acid having a free thiol or amino group is present in the P-loop; (c) labeling the kinase of interest with a thiol- or amino-reactive fluorophore sensitive to polarity changes in its environment, a thiol-reactive spin label, an isotope or an isotope-enriched thiol- or amino-reactive label such that said fluorophore, spin label, isotope or isotope-enriched label does not inhibit the catalytic activity and/or does not interfere with the stability of the kinase; (d) contacting the kinase obtained in step (c) with a known inhibitor of said kinase; and (e) recording the fluorescence emission signal at one or more wavelengths or a spectrum of said fluorescently labeled kinase of step (c) and (d) upon excitation or (e)' recording the EPR or NMR spectra of said spin-labeled kinase of step (c) and (d); and (f) comparing the fluorescence emission signal at one or more wavelengths or the spectrum recorded in step (e) or the EPR or NMR spectra recorded in step (e)'; wherein a difference in the fluorescence intensity at at least one wavelength, preferably the emission maximum and/or a shift in the fluorescence emission wavelength in the spectra of said fluorescently labeled kinase obtained in step (e), or an alteration in the EPR or NMR spectra of said spin-labeled or isotope-labeled kinase obtained in step (e)' indicates that the kinase is suitable for the screening for kinase inhibitors.

Adapted to a high-throughput format, multiple kinases or differently labeled variations of the same kinase can be screened.

The term "unsuitable position" in accordance with the present invention denotes a position in the P-loop which was shown to be not suitable for an amino acid labeled according to the invention. This can be due to a decreased sensitivity of the label to changes in its environment or due to predictions based on structural considerations that said position would result in a kinase with a label with decreased sensitivity. The term also encompasses amino acids positioned at a potentially suitable position, wherein a different position is deemed more appropriate. As soon as the number of amino acids having a free thiol or amino group in the P-loop exceeds one, amino acids deemed as unsuitable should be mutated.

Mutating an amino acid includes replacing or deleting said amino acid with another amino acid, provided that said mutation does not result in an inhibited catalytic activity or an interference with the stability of the resulting kinase. Step (b) is carried out if no amino acid having a free thiol or amino group is present in the P-loop of said kinase of interest. The amino acid which is inserted or which replaces another amino acid has to have a free thiol or amino group in order to be labeled.

In a preferred embodiment of the methods of the present invention, the kinase inhibitor binds either exclusively to the allosteric site adjacent to the ATP binding site of the kinase or extends from the allosteric site into the ATP site. These types of inhibitors are also called Type III or Type II inhibitors, respectively. They bind to kinases with higher specificity as compared to Type I inhibitors which bind to the ATP-pocket of the kinase, which is highly conserved in structure among all kinases.

As demonstrated in the examples, the present invention provides means to differentiate between ATP-competitive and non-ATP-competitive inhibitors, enabling for a rapid election of specific inhibitors. The invention is designed to detect the movement of the P-loop of the kinase, which is caused by movement of the activation loop and is therefore sensitive to all Type II and Type III inhibitors. Furthermore, certain Type I inhibitors exerting a specific binding mode (see above) or directly interact with the P-loop with the kinase in the DFG-in conformation are detected. Only measurement of the fluorescence change over time (i.e. not an endpoint measurement) resulting in the determination of the rate of binding can allow Type I inhibitors to be distinguished from Type II and Type III inhibitors. As presented in one of the examples below, detected ATP-competitive inhibitors produce an instantaneous fluorescence change (typically < 5 sec) while Type II and Type III inhibitors bind much slower (seconds to several minutes).

In another preferred embodiment of the kinase or the methods of the present invention, the kinase is labeled at a cysteine naturally present or introduced into the P-loop.

The abundance of cysteines in proteins is usually very low, so that a kinase of the invention can be prepared in a straightforward manner by replacing an amino acid in the P-loop with cysteine and optionally replacing solvent-exposed cysteines with other amino acids. Amino acids containing reactive amines, such as histidine, arginine or lysine or derivatives thereof, are much more abundant and are readily found at the protein surface where they are in contact with the surrounding solvent. Thus, it is preferable to use thiol-reactive labels which can specifically react with an introduced cysteine.

In a more preferred embodiment, the method of screening for kinase inhibitors or the method of generating mutated kinases further comprises step (c1) measuring a fluorescence intensity ratio of two wavelengths recorded in step (c) and obtaining the ratio of the normalized intensity change to the average intensity change ($\Delta I_{std}$).

Additionally or alternatively, the maximum standard intensity change ($\Delta R_{max}$) between a kinase labeled according to the invention with inhibitor bound and one without inhibitor may be assessed. A candidate compound is considered a kinase inhibitor or the fluorescent- labeled kinase is considered suitable for the screening for kinase inhibitors if ($\Delta I_{std}$) is > 0.25, and/or ($\Delta R_{max}$) is > 0.75 and the Z-factor is > 0.5. This embodiment relates to the extension of the methods of the present invention to high-throughput scale as described above.

$\Delta I_{std}$ is the ratio of normalized intensity change to average intensity of the fluorescence emission. According to de Lorimier et al. (2002), $\Delta I_{std}$ is one of the most important criteria for characterizing a fluorescent protein conjugate as suitable for sensitive fluorescence spectroscopy. Ideally, the $\Delta I_{std}$ should have a value > 0.25 and is calculated by:

$$\Delta I_{std} = \left| \frac{2(I_1(\lambda_{std}) - I_2(\lambda_{std}))}{I_1(\lambda_{std}) + I_2(\lambda_{std})} \right|$$

where $\lambda_{std} = (\lambda_{max, \text{unbound}} + \lambda_{max, \text{saturated}})/2$ and $I_1$, $I_2$ are the fluorescence intensities at $\lambda_{std}$ of each spectrum respectively.

$\Delta R_{max}$ is the maximum standard intensity change of the fluorescence emission between saturated and unsaturated kinase. According to (de Lorimier et al., 2002), $\Delta R_{max}$ is another important criteria for characterizing a fluorescent protein conjugate as suitable for sensitive fluorescence spectroscopy. Ideally, the $\Delta R_{max}$ should have a value > 1.25 and is calculated by:

$$\Delta R = \left| \frac{^0A_1}{^0A_2} - \frac{^\infty A_1}{^\infty A_2} \right|$$

where $°A_1$, $°A_2$ are the areas in the absence of ligand, and $^∞A_1$, $^∞A_2$ are the areas in the presence of saturating ligand. A computer program can be used to enumerate $\Delta R$ for all possible pairs of wavelength bands in the two spectra, to identify the optimal sensing condition, defined as the maximum value of $\Delta R$.

The Z-factor is a statistical measure of the quality or power of a high-throughput screening (HTS) assay. In an HTS campaign, large numbers of single measurements of unknown samples are compared to well established positive and negative control samples to determine which, if any, of the single measurements are significantly different from the negative control. Prior to starting a large screening campaign, much work is done to assess the quality of an assay on a smaller scale, and predict if the assay would be useful in a high-throughput setting. The Z-factor predicts if useful data could be expected if the assay were scaled up to millions of samples. The Z-factor is calculated by:

$$Zfactor = 1 - \frac{3 \times (\sigma_p + \sigma_n)}{|\mu_p - \mu_n|}$$

wherein both the mean ($\mu$) and standard deviation ($\sigma$) of both the positive (p) and negative (n) controls ($\mu_p$,$\sigma_p$,$\mu_n$,$\sigma_n$, respectively) are taken into account.

The measurement of $\Delta I_{std}$ and $\Delta R_{max}$ as well as the determination of the Z-factor may prove useful in determining whether the label chosen is suitable in the screening for inhibitors. De Lorimier discusses that the measured kinetics and Kd obtained with a fluorescent tagged protein will depend on the protein, the ligand and the fluorophore used. Therefore, the same inhibitor binding to the same kinase could give different Kd values depending on the label used. The determination of the above values might indicate whether the label chosen is appropriate or whether a different label should be used.

In a further preferred embodiment, the fluorophore or spin-label is not located at or adjacent to phosphorylation sites known or predicted to exist in the labeled kinase. This ensures that the labeling does not interfere with the dynamics of the P-loop or the normal activity and regulation of the kinase which is largely affected by phosphorylation and dephosphorylation.

In another preferred embodiment, said candidate amino acid in the P-loop is identified based on structural and/or sequence data available for said kinase.

For some kinases, structural data, e.g. in the form of crystal or NMR structures is available, wherein the kinase is captured in the activated and/or inactivated state. If such data is available for a kinase, this facilitates the choice of the amino acid position in the P-loop to be replaced for labeling purposes. The actual choice is based on which residue tends to exhibit the most movement in the P-loop in response to ligand binding and/or conformational changes in the position preceding the third Gly of the G-X-G-X-X-G motif, which is most often a Tyr or Phe. If this residue is a Tyr and is known to be phosphorylated in a particular kinase, which is rarely the case, labeling of this position will likely disrupt kinase activity and makes this position unsuitable in the present invention. Similarly, contacts of the amino acid in said position with other amino acids are also examined. If said contacts are deemed essential for the catalytic activity or stability of the kinase, the position is in most cases not suitable for replacement. Additionally, the choice is based on the distance which a particular amino acid will move as the protein changes conformation such that greater distances increase the chance that an environmental change will be detected. However, although distance moved is an indicator of whether a particular position may be useful for labeling, it is the actual change in environment which will correlate directly with the observed changes detected by the attached label.

In a preferred embodiment, the methods of the present invention relating to screening for inhibitors, determining kinetic parameters such as association and dissociation and generating a mutated kinase are combined to obtain a straightforward methodology to obtain specific inhibitors for different kinases. In this regard, any preferred embodiment of a method of the invention may be combined with embodiments of other methods of the invention. In a more preferred embodiment of this aspect, an initial screen is carried out using the method of high-throughput screening for kinase inhibitors, followed by a screen using a wide range of concentrations of inhibitors as described above with the method of the invention for determining the kinetics of ligand binding and/or association or dissociation. The latter step is carried out, inter alia, to get an indication of the Kd and/or Ka value. This step is again repeated by carrying out measurements with a more focused concentration range for more precise measurements of the Kd or Ka. These measurements may be carried out either as a titration series with the cuvette approach and/or real-time kinetic measurements in cuvettes ($k_{on}$ and $k_{off}$) to further characterize each inhibitor. Optionally, this sequence of methods is transferred to other kinases or the same kinase labeled differently. This embodiment is designed to enable for high-throughput screening to screen for and characterize a high number of inhibitors in multiple kinases or differently labeled variations of the same kinase.

More specifically, such a combined method is a method for identifying a kinase inhibitor binding either partially or fully to the allosteric site adjacent to the ATP binding site of a kinase comprising (a) screening for an inhibitor according to the method of screening for kinase inhibitors of the invention, and (b) determining the rate constant of an inhibitor identified in step (a) to a kinase, wherein a rate of binding of <0.140 s$^{-1}$ determined in step (b) indicates that the kinase inhibitor identified binds either partially or fully to the allosteric site adjacent to the ATP binding site of the kinase. Rate constants of >0.140 s$^{-1}$ indicate that the kinase inhibitor identified binds in the ATP binding site and does not extend into the adjacent allosteric site. The rate constant is correlated to the reaction time (rate of binding) $t_{1/2}$: $t_{1/2} = \ln(2)/k_{obs}$. Accordingly, a rate constant ($k_{obs}$) of <0.140 s$^{-1}$ corresponds to a reaction time $t_{1/2}$ of >5 s.

The rate constant or rate of binding is preferably determined using the properties of the labeled kinase of the invention. For example, the kinase of the invention can be contacted with an inhibitor and, depending on the label, the fluorescence emission signal of a fluorescently labeled kinase at one or more wavelengths or the electron paramagnetic resonance or nuclear magnetic resonance spectra of a spin-labeled or isotope-labeled kinase can be recorded over time. This corresponds to steps (a) to (c) of the method of determining the kinetics of ligand binding and/or of association or dissociation of a kinase inhibitor of the invention or steps (a) and (b) of the method of determining the dissociation or association of a kinase inhibitor of the invention. In case the rate of binding, i.e. the measurable changes in fluorescence or in the NMR or EPR spectra, is more than 5 seconds after application of the inhibitor, this indicates that the inhibitor is a type II or type III inhibitor.

In another preferred embodiment of the method for screening of kinase inhibitors, the method further comprises (subsequently) optimizing the pharmacological properties of a candidate compound identified as inhibitor of said kinase.

Methods for the optimization of the pharmacological properties of compounds identified in screens, generally referred to as lead compounds, are known in the art and comprise a method of modifying a compound identified as a lead compound to achieve: (a) modified site of action, spectrum of activity, organ specificity, and/or (b) improved potency, and/or (c) decreased toxicity (improved therapeutic index), and/or (d) decreased side effects, and/or (e) modified onset of therapeutic action, duration of effect, and/or (f) modified pharmacokinetic parameters (absorption, distribution, metabolism and excretion), and/or (g) modified physico-chemical parameters (solubility, hygroscopicity, color, taste, odor, stability, state), and/or (h) improved general specificity, organ/tissue specificity, and/or (i) optimized application form and route by a. esterification of carboxyl groups, or b. esterification of hydroxyl groups with carboxylic acids, or c. esterification of hydroxyl groups to, e.g. phosphates, pyrophosphates or sulfates or hemi-succinates, or d. formation of pharmaceutically acceptable salts, or e. formation of pharmaceutically acceptable complexes, or f. synthesis of pharmacologically active polymers, or g. introduction of hydrophilic moieties, or h. introduction/exchange of substituents on aromates or side chains, change of substituent pattern, or i. modification by introduction of isosteric or bioisosteric moieties, or j. synthesis of homologous compounds, or k. introduction of branched side chains, or I. conversion of alkyl substituents to cyclic analogues, or m. derivatization of hydroxyl group to ketales, acetales, or n. N-acetylation to amides, phenylcarbamates, or o. synthesis of Mannich bases, imines, or p. transformation of ketones or aldehydes to Schiffs bases, oximes, acetales, ketales, enolesters, oxazolidines, thiazolidines or combinations thereof.

The various steps recited above are generally known in the art. They include or rely on quantitative structure-action relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 140(8), 813-823, 2000).

The figures show

**Figure 1. Schematic representation of changes in P-loop and activation loop conformations triggered by ligand binding in p38$\alpha$.** a) Structurally important regions (P-loop; helix C; hinge region) of the active kinase domain (DFG-in) are labeled. b) Mobility of the activation loop in resonance to the activation loop. Type II/III inhibitors occupy a site that is present only in the DFG-out kinase conformation. This allosteric pocket is flanked by the DFG-motif and helix C. Type II/III inhibitor binding to the allosteric site causes a conformational change in the activation loop (black) and allows the P-loop (black) to adopt a more extended conformation. c) A Cys residue was mutated into the P-loop of p38$\alpha$ for subsequent labeling with an environmentally-sensitive fluorophore (large sphere) to generate a sensitive P-loop binding assay. Active (DFG-in) and inactive (DFG-out) kinase conformations are in equilibrium and result from structural changes in the activation loop. Structural changes of the activation loop are transmitted to the P-loop through a hydrophobic interface and change the chemical environment the fluorophore attached to the P-loop. Type I inhibitor (surface behind the large sphere in the left panel) binds to the hinge region of the active kinase (DFG-in) (left panel). In this particular case the P-loop folds over to directly interact with the inhibitor. In absence of ligands from active kinase (DFG-in) the P-loop adopts a more extended conformation (middle panel). Type II/III inhibitors (surface below large sphere in the right panel) bind to inactive (DFG-out) kinase conformations.

**Figure 2: Real-time and endpoint fluorescence measurements using ac-p38$\alpha$ labeled on the glycine-rich loop**. Acrylodan emission at 475 nm decreases upon binding of BIRB-796 resulting in a red-shift (shift to longer wavelength) of the maximum emission wavelength in the bound state (A). Endpoint equilibrium measurements can be made to directly obtain the $K_d$. Ratiometric fluorescence data (R = 512 nm/475 nm) were plotted on a logarithmic scale of inhibitor concentration to obtain the $K_d$ (B). Ratiometric fluorescence data (R = 445 nm/475 nm) can also be used to obtain the Kd (*data not shown*). Fluorescence traces can also be measured in real-time at a single wavelength (475 nm) to determine various kinetic rate constants. The fluorescence decay resulting from the addition of different amounts of BIRB-796 (large arrow) was fit (gray lines) to a first-order decay function to obtain $k_{obs}$ (C). Experimentally determined $k_{obs}$ values can then be plotted to determine $k_{on}$ for BIRB-796 any ligand. Extraction of BIRB-796 from ac-p38$\alpha$ using an excess of unlabeled p38$\alpha$ allowed direct determination of $k_{off}$ which was also fit (gray lines) to a first-order function (D). The data presented above are representative of a typical set of experiments carried out for BIRB-796 using ac-p38$\alpha$ labeled on the glycine-rich loop.

**Figure 3. Fluorescence characterization and response of P-loop labeled p38$\alpha$ to different inhibitor types.** The structures of various inhibitor types (Type I, II or III) are shown in addition to the structures of Scios-469 and **RL40,** two hits identified in a compound screen. The P-loop was labeled by covalently modifying Y35C of p38$\alpha$ with the thiol-reactive fluorophore acrylodan and the changing fluorescence properties were examined upon binding of known DFG-out and DFG-in binders of p38$\alpha$. All values for $\Delta R_{max}$ and $\Delta I_{std}$ which meet the criteria deemed ideal fluorophore-protein conjugates (deLorimier et al. 2003) appear in **bold** text. In the case of traditional DFG-out binders (Type II and Type III inhibitors) or some Type I inhibitors which directly interact with the P-loop, acrylodan exhibits a large emission shift but there is an increase in emission at ~512 nm relative to ~475 nm, still allowing reliable binding curves to be measured despite the suboptimal $\Delta R_{max}$. However, superior $\Delta I_{std}$ values were obtained in the case of these same types of inhibitors. SB203580 is a Type I inhibitor of p38$\alpha$ known to stabilize the DFG-out conformation (as disclosed in EP 08 02 0341). Dasatinib binds to the hinge region of the kinase and does not interact with the DFG motif or the P-loop (Tokarski et al., 2006) and was not detected (ND) by this assay system. Type I inhibitors such as SB20358 and DFG-out binding Type II (BIRB-796) and III (**RL36**) inhibitors were sensitively detected, allowing for Kd and kinetic measurements. The kinetic measurements allow for the discrimination of Type I ligands, which bind very rapidly (< 2sec) from Type II/III ligands, which are known to bind slowly to p38$\alpha$ (Pargellis et al. 2003). Two such ligands, **RL40** and Scios-469, were detected in a screening initiative. Protein X-ray crystallography was later employed to understand the structural details behind the detection of these two ligands. *[Note: \*$\Delta I_{std}$ was calculated using emission intensities at 445 and 475 nm in presence and absence of ligand (R = 445/475 nm is most optimal to detect ligand binding); \*\* $\Delta Rmax$ was calculated using emission intensities at 475 and 512 nm in presence and absence of ligand (R = 512/475 nm is most optimal to discriminate binding mode).]*

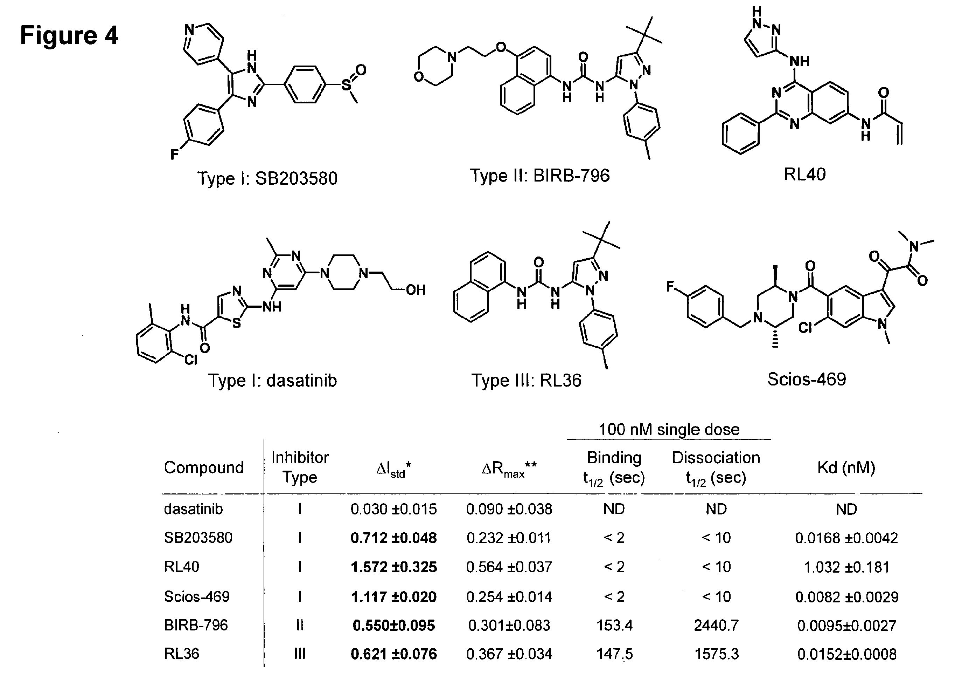

**Figure 4: Real-time and endpoint fluorescence measurements of a Type III and Type I ligand using glycine-rich loop labeled ac-p38$\alpha$.** Acrylodan emission at 475 nm decreases upon binding of the Type III ligand RL36 resulting in a red-shift of the maximum emission wavelength in the bound state (a). Similar but more intense changes were observed for SB203580, a Type I ligand known to stabilize the DFG-out conformation of p38$\alpha$ by interacting with the P-loop (b). Since Type III and Type II ligands (see Fig. 2) trigger a change in the activation loop conformation which results in an associated structural rearrangement of the glycine-rich loop (see **Fig. 1a),** both spectral shape and loss of intensity change similarly for both inhibitor types. Fluorescence traces were measured in real-time at a single wavelength (475 nm) to determine the rate of ligand binding and dissociation. The fluorescence decay resulting from the addition (black arrow) of 100 nM **RL36** was fit (gray lines) to a first-order decay function to obtain $k_{obs,on}$ ((a) center). Type I ligands such as SB203580 typically bind < 2 sec ((b) center) and accurate curve fitting is not possible without the use of stop-flow fluorescence spectroscopy to increase the time resolution of the measurement. Extraction of each inhibitor from ac-p38$\alpha$ was accomplished by adding an excess of unlabeled p38$\alpha$ to the same sample (white arrow). Since it is known that the $k_{off}$ is significantly slower than $k_{obs,on}$ for all inhibitor types, it was possible to determine the $k_{off}$ for each inhibitor by fitting (gray lines) the fluorescence increase to a first-order function. Ratiometric fluorescence data (R = 512 nm/475 nm) were plotted on a logarithmic scale of inhibitor concentration to obtain the $K_d$ for **RL36** ((a) right) and SB203580 ((b) right). The Type II inhibitor imatinib does not bind to p38$\alpha$ and served as a negative control for **RL36** ((a) right, black squares). The Type I inhibitor dasatinib binds to p38$\alpha$ but does not interact with the glycine-rich loop and served as a negative control for SB203580 ((b) right, black squares). The data presented above are representative of a typical set of experiments carried out using ac-p38$\alpha$ labeled on the glycine-rich loop.

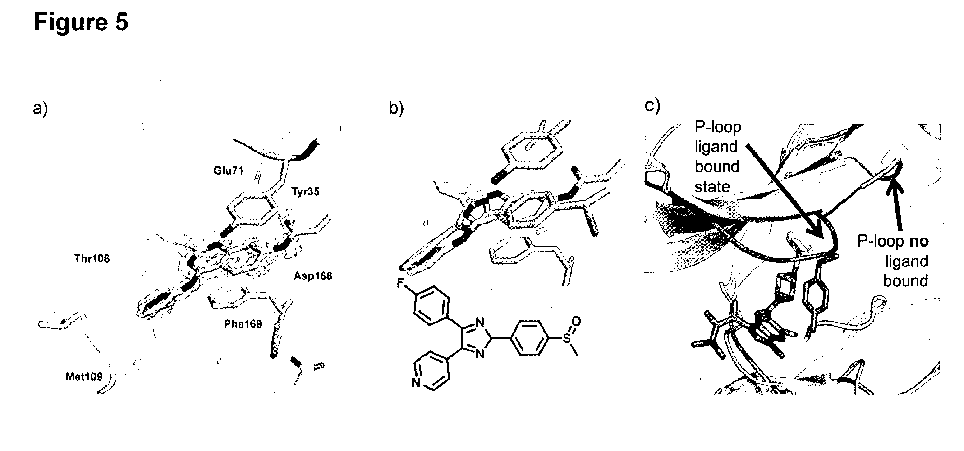

**Figure 5. Crystal structures of RL40 and Scios-469 confirm movement of the P-loop**. The structure of **RL40** in complex with p38$\alpha$ (A) reveals a unique and unexpected binding mode analogous to that observed in the structure of SB203580 reported previously (EP 08 02 0341) in which the ligand interacts with the P-loop by forming a unique $\pi$-$\pi$ stacking with the Phe side chain of the DFG motif. The result of this interaction is the stabilization of the DFG-out conformation. An overlay of the structures for SB203580 and **RL40** in p38$\alpha$ revelas that the aromativ cores of both inhibitors nicely overlay and form the same type of stacking interactions with the P-loop and activation-loop. Analogs of **RL40** are typically observed binding to the hinge region of kinases and do not interact with the P-loop (Pierce et al., 2005), thus highlighting the benefit of using P-loop labeled kinases to enrich for ligands which take advantage of these unique binding modes. Additionally, the P-loop labeled kinase assay strongly detected the binding of Scios-469. We co-crystallized Scios-469 with wild-type p38$\alpha$ and solved the structure to a resolution of 2.5 A (c). We observed a dramatic movement in the P-loop when compared to the apo structure of p38$\alpha$. This movement is induced and stabilized by stacking interactions of the P-loop Tyr35 (the chosen labeling position for the assay) with hydrophobic features of the compound. This provides an example of how the P-loop labeled kinase assay can sensitively detect some Type I ligands which directly alter the conformation of the P-loop.

The examples illustrate the invention

### Example 1: Selection of a Suitable Kinase

We chose to work with p38$\alpha$ to develop this assay for the following reasons: i) the abundance available of structural information, ii) the availability of crystal structures in both its active and inactive conformations (Figure 1A.) and iii) the availability of tight binding Type II & III allosteric inhibitors. In the first step, the crystal structures of p38$\alpha$ were closely examined to identify suitable fluorophore attachment sites that would detect allosteric binders. Candidate residues for this mutation must be solvent exposed to enable the attachment of a fluorophore by Michael addition, and exhibit significant movement upon ligand binding. Care was also taken to not choose residues that are critical to maintaining protein stability, catalytic activity or residues in the vicinity of known phosphorylation sites.
A position in P loop was selected and subsequently mutated into a cysteine residue (Figure 1B.,C.). Acrylodan was selected as the fluorophore due to its relatively small size (comparable to a tryptophan side chain), its high sensitivity to polarity changes, its commercial availability and relatively low price. Acrylodan is also known to produce a robust response and should detect movements of the activation loop upon binding of allosteric inhibitors (Figure 1D .). Before labeling the protein, it was necessary to reduce the chances of fluorophore attachment to any other solvent exposed cysteine residues. Again, structural information was used to locate 4 reduced cysteine residues in p38$\alpha$. Two of these cysteine are buried within the protein while the other two were solvent-exposed and conservatively mutated into serine. Lastly, a F327L mutation was incorporated to partially activate (Askari et al., 2007: Avitzour et al., 2007) the acrylodan-labeled p38$\alpha$ (ac-p38$\alpha$) for use in enzyme activity assays, if desired, but is not necessary for functionality of the assay itself.

### Example 2: Protein Labeling and Fluorescence Characterization

Protein labeling

A p38$\alpha$ construct containing 4 total mutations (2 cysteine $\rightarrow$ serine, and the introduction of a cysteine for labeling) was transformed into the BL21(DE3) *E. coli* strain, overexpressed, purified by affinity, anion exchange and size exclusion chromatography and the pure protein was subsequently used for labeling. Protein and free acrylodan were combined at a 1:1.5 ratio and allowed to react in the dark overnight at 4˚C. The conjugated protein (ac-p38$\alpha$) was concentrated, aliquoted and frozen at -20˚C. Mono-labeling of 100% of the protein was verified by ESI-MS. Confirmation of the correctly labeled cysteine is currently being performed by analyzing the tryptic fragments of unlabelled and labeled p38$\alpha$ following a combination of HPLC and ESI-MS or MALDI.

Fluorescence characterization

Following labeling, the fluorescent properties of the probe were characterized and initial experiments were carried out using various derivatives of the pyrazolourea Type II allosteric inhibitor, BIRB-796 (Pargellis et al., 2002; Dumas et al., 2000 (a and b); Moss et al., 2007; Regan et al., 2002; Regan et al., 2003). The ac-p38$\alpha$ protein labeled on the P-loop shows a modest red-shift from 475 nm to 512 nm with ligand binding (Figure 2A). Measuring a ratio of two wavelengths (R = 512 nm / 475 nm) allows the possibility of eliminating dilution errors between different samples (Figure 2B). Using these two wavelengths, average Z-factors of 0.53 $\pm$ 0.04 can be calculated. Similarly, a small maxima present at 445 nm is relatively insensitive to ligand binding, and as a result, the ratio of two wavelength (R = 445 nm / 475 nm) can also be used for this purpose. Using these two wavelengths, average Z-factors of 0.85 $\pm$ 0.05 can be calculated. A large change at 475 nm also allows for the possibility of making single-wavelength kinetic measurements (Figure 2C-D).
Using the wavelengths 512 nm and 475 nm, the normalized intensity change compared to average intensity ($\Delta I_{std}$) was determined to range between 0.55 - 1.57 for all detected inhibitor types and the maximum standard intensity change ($\Delta R_{max}$) between saturated and unsaturated ac-p38$\alpha$ ranged between 0.23 - 0.56 for all detected inhibitor types. These are two of the most important criteria for fluorescence spectroscopy (de Lorimier et al., 2002). The $\Delta I_{std}$ values together with the determined Z factors for the two different ratiometric readouts characterize this as a suitable probe for use in fluorescence assays.

**Example 3: Kinase Expression & Purification**

The p38α construct was cloned into a pOPINE vector and was transformed as an N-terminal His-tag construct with Precision Protease cleavage site into BL21(DE3) *E. coli.* Cultures were grown at 37˚C until an OD600 of 0.6, cooled in 30 min to RT and then induced with 1 mM IPTG for overnight (~20 hrs) expression at 18˚C while shaking at 160 rpm. Cells were lysed in Buffer A (50 mM Tris pH 8.0, 500 mM NaCl + 5% glycerol + 25 mM imidazole) and loaded onto a 30 mL Ni-column (self-packed), washed with 3 CV of Ni Buffer A and then eluted with a 0-50% linear gradient using Ni Buffer B (Ni Buffer A + 500 mM imidazole) over 2 CV. The protein was cleaved by incubating with PreScission Protease (50 μg/mL final concentration) in a 12-30 mL capacity 10-MWCO dialysis cassette (Thermo Scientific) overnight at 4˚C in Dialysis Buffer (50 mM Tris pH 7.5, 5% glycerol, 150 mM NaCl, 1 mM EDTA, 1 mM DTT). The protein was then centrifuged for 15 min at ~13,000 rpm to remove any precipitate that may have formed during the cleavage step. The supernatant was then taken and diluted at least 4-fold in Anion Buffer A (50 mM Tris pH 7.4, 5% glycerol, 50 mM NaCl, 1 mM DTT) and loaded onto a 1 mL Sepharose Q FF column (GE Healthcare) and washed with 10 CV of Anion Buffer A. The protein was eluted with a 0-100% linear gradient of Anion Buffer B (Anion Buffer A + 600 mM NaCl) over 20 CV. The protein was pooled and concentrated down to 2 mL and passed through a Sephadex HiLoad 26/60 Superdex 75 column equilibrated with Size Exclusion Buffer (20 mM Tris pH 7.4, 5% glycerol, 200 mM NaCl, 1 mM DTT) at a rate of 2 mL/min. The eluted protein was then concentrated to ~10 mg/mL, aliquoted and frozen at -80oC.

**Example 4: Real-time and endpoint fluorescence measurements using ac-p38α labeled on the glycine-rich loop enables detection of Type II/III inhibitors and some Type I inhibitors.**

Upon binding of both Type II (BIRB-796) and Type III (RL36) inhibitors, acrylodan emission at 475 nm decreases resulting in a red-shift of the maximum emission wavelength in the bound state (Figure 2A and 3Aleft panel). At equilibrium, the emission spectra of P-loop labeled p38α changes such that the emission intensities at 512 nm and 475 nm are nearly equal (i.e. R = 512/475 nm usually has a value of ~ 1.0). Using this ratiometric fluorescence output, endpoint equilibrium measurements can be made to directly obtain the $K_d$ of these ligands (Figure 2B and 3Aright) by plotting the fluorescence data against a logarithmic scale of inhibitor concentration. This response is characteristic of any Type II or III inhibitor which occupies the allosteric site adjacent to the ATP-site and requires a conformational change in the activation loop. As described above, this conformational change induces a characteristic change in the P-loop which alters the fluorescence of acrylodan in a specific manner. Type I ligands known to stabilize the DFG-out conformation by stacking between the P-loop and the Phe of the DFG motif are also sensitively detected and induce even larger intensity losses at 475 nm when compared to Type II/III ligands (Figure 4Bleft).

The discrimination of such Type I ligands from Type II or III ligands is easily accomplished by examining the binding kinetics of each ligand. Kinetic measurements are made by monitoring the decrease in emission intensity at a single wavelength (475 nm) upon addition of ligand to a suspension of the labelled kinase in buffer. In the case of Type II inhibitors such as BIRB-796 (Figure 2C) and Type III inhibitors such as RL36 (Figure 3amiddle), the kinetics of binding is significantly slower than that of Type I inhibitors, regardless of whether the Type I inhibitor stabilizes the DFG-in or DFG-out conformation (see kinetic values in Figure 4). The slower fluorescence decays resulting from the addition of different Type II or III inhibitors can be easily fit to a first-order decay function to obtain $k_{obs}$ (Figure 3a - middle). Experimentally determined $k_{obs}$ values can then be plotted to determine $k_{on}$ for BIRB-796 any ligand as described in EP 08 01 3340 and EP 08 02 0341. The fluorescence decays of Type I inhibitor binding are too fast to fit accurately without the use of stopped-flow fluorescence spectroscopy. Extraction of inhibitors from ac-p38α using an excess of unlabeled p38α results in an upward change in the fluorescence intensity at 475 nm which was also fit to a first-order function fluorescence to allow direct determination of $k_{off}$. Regardless of inhibitor type, the rate of dissociation of the ligand from the protein is always slower than the rate of binding, a well-known observation, particularly for p38α (Pargellis et al. 2003).

With respect to the determined Kd values for all detected ligand types, the affinities are in very close agreement with those determined and published elsewhere using other methods (Pargellis et al. 2003, EP 08 01 3340 and EP 08 02 0341). The close agreement between the Kd values and the kinetic trends reported using the P-loop labelled assay and other methods validates the assay system. Additionally, a Type II ligand known to not bind to p38α (imatinib) and a Type I inhibitor (dasatinib) that potently inhibits p38α (Karaman et al 2008), but does not interact with the DFG motif or the P-loop, were not detected by the assay and could be used as negative controls (Figure 3a right; Figure 3b right).

**Example 5: Crystal structures of RL40 and Scios-469 confirm movement of the P-loop.**

We screened several compounds against P-loop labeled p38α and identified **RL40** and Scios-469 as being sensitively detected in the assay. To understand the structural details which explain the detection of these ligands, we co-crystallized **RL40** and Scios-469 with wild-type p38α. The structure of **RL40** in complex with p38α (Figure 5A) reveals a unique and unexpected binding mode which is analogous to that observed in the structure of SB203580 reported previously (EP 08 01 3340 and EP 08 02 0341). The ligand interacts with the P-loop by forming a unique π-π stacking with the Phe of the DFG motif and thereby stabilizes the DFG-out conformation and provides a rational explanation for ist detection in the assay. An overlay of this structure with that of the SB203580-p38α complex (Figure 5B) reveals that features of both inhibitors nicely overlay and provides insight into future chemical modifications to improve affinity The binding mode was unexpected since analogs of **RL40** typically bind to the hinge region of kinases and adopt a completely different orientation within the ATP binding site. Furthermore, they typically do not interact with the P-loop, as was demonstrated elsewhere for one such analog bound to GSK3β (Pierce et al., 2005). These findings highlight the benefit of using P-loop labeled kinases to enrich for ligands which take advantage of this unique binding modes such as **RL40** and SB203580.

Additionally, the P-loop labeled kinase assay strongly detected the binding of Scios-469. We co-crystallized Scios-469 with wild-type p38α (Figure 5C) and observed that this ligand is a Type I inhibitor which induces a dramatic movement in the P-loop. More importantly, the kinase is in the DFG-in conformation, suggesting that the compound is detected solely due to this interaction. This P-loop movement is induced and stabilized by the π-π stacking of the P-loop Tyr35 (the chosen labeling position for the assay) with hydrophobic features of the compound. Aside from detected all DFG-out binders, Scios-469 provides an example of how the P-loop labeled kinase assay can sensitively detect some Type I ligands which directly alter the conformation of the P-loop.

**References**

Annis, D. A., Nazef, N., Chuang, C. C., Scott, M. P., and Nash, H. M. (2004) A general technique to rank protein-ligand binding affinities and determine allosteric versus direct binding site competition in compound mixtures, Journal of the American Chemical Society 126, 15495-15503.R. T. Aimes, W. Hemmer, S. S. Taylor, Biochemistry 2000, 39, 8325.

B. Apsel, J. A. Blair, B. Gonzalez, T. M. Nazif, M. E. Feldman, B. Aizenstein, R. Hoffman, R. L. Williams, K. M. Shokat, Z. A. Knight, Nat Chem Biol 2008, 4, 691.

A. C. Backes, B. Zech, B. Felber, B. Klebl, G. Müller, Expert Opin Drug Discovery 2008, 3, 1409.

A. C. Backes, B. Zech, B. Felber, B. Klebl, G. Müller, Expert Opin Drug Discovery 2008, 3, 1427.

J. A. Blair, D. Rauh, C. Kung, C. H. Yun, Q. W. Fan, H. Rode, C. Zhang, M. J. Eck, W. A. Weiss, K. M. Shokat, Nat Chem Biol 2007, 3, 229.

R. A. Copeland, D. L. Pompliano, T. D. Meek, Nat Rev Drug Discov 2006, 5, 730. Dar, A. C., Lopez, M. S., and Shokat, K. M., Small Molecule Recognition of c-Src via the Imatinib-Binding Conformation. Chem Biol 15 (10), 1015 (2008).DeLano, W.L., The PyMOL Molecular Graphics System. http:llwww.pymol.org (2002). de Lorimier, R. M., Smith, J. J., Dwyer, M. A., Looger, L. L., Sali, K. M., Paavola, C. D., Rizk, S. S., Sadigov, S., Conrad, D. W., Loew, L., and Hellinga, H. W. (2002) Construction of a fluorescent biosensor family, Protein Sci 11, 2655-2675.Dumas, J., Hatoum-Mokdad, H., Sibley, R., Riedl, B., Scott, W. J., Monahan, M. K., Lowinger, T. B., Brennan, C., Natero, R., Turner, T., Johnson, J. S., Schoenleber, R., Bhargava, A., Wilhelm, S. M., Housley, T. J., Ranges, G. E., and Shrikhande, A. (2000) 1-Phenyl-5-pyrazolyl ureas: potent and selective p38 kinase inhibitors, Bioorganic & medicinal chemistry letters 10, 2051-2054. Emsley, P. and Cowtan, K., Coot: model-building tools for molecular graphics. Acta Crystallogr D Biol Crystallogr 60 (Pt 12 Pt 1), 2126 (2004).

M. J. Garnett, R. Marais, Cancer Cell 2004, 6, 313.

Gauglitz, G. and Vo-Dinh, T. (2003). Handbook of spectroscopy. Wiley-VCH.

Gschwind, A., Fischer, O. M. and Ullrich, A., The discovery of receptor tyrosine kinases: targets for cancer therapy. Nat Rev Cancer 4 (5), 361 (2004).S. K. Hanks, T. Hunter, FASEB J 1995, 9, 576.

Hibbs, R. E., Talley, T. T., and Taylor, P. (2004) Acrylodan-conjugated cysteine side chains reveal conformational state and ligand site locations of the acetylcholine-binding protein, The Journal of biological chemistry 279, 28483-28491.

Johnson, L. N., Noble, M. E. M. and Owen, D. J. (1996) Active and inactive protein kinases : structural basis for regulation. Cell 85, 149-158.Johnson, L. N. and Lewis, R. E. (2001). Structural basis for control by phosphorylation. Chem Rev. 2001 Aug;101(8):2209-42.

Kabsch, W., Automatic processing of rotation diffraction data from crystals of initially unknown symmetry and cell constants.. J. Appl. Cryst. 26, 795 (1993).

Lakowicz, J. R. (1999). Principles of Fluorescence Spectroscopy. Kluwer Academic / Plenum Publishers

Laskowski, R. A., MacArthur, M. W., Moss, D. S., and Thornton, J. M. , PROCHECK: a program to check the stereochemical quality of protein structures. J Appl Cryst 26, 283 (1993).

Larkin, M. A. et al., Clustal W and Clustal X version 2.0. Bioinformatics 23 (21), 2947 (2007).

Levinson, N. M. et al., A Src-like inactive conformation in the abl tyrosine kinase domain. PLoS Biol 4 (5), e144 (2006).

Liu, Y. and Gray, N. S., Rational design of inhibitors that bind to inactive kinase conformations. Nat Chem Biol 2 (7), 358 (2006).M. Mapelli, L. Massimiliano, C. Crovace, M. A. Seeliger, L. H. Tsai, L. Meijer, A. Musacchio, J Med Chem 2005, 48, 671.

Michalczyk, A. et al., Structural insights into how irreversible inhibitors can overcome drug resistance in EGFR. Bioorg Med Chem 16 (7), 3482 (2008).

Murshudov, G. N., Vagin, A. A., and Dodson, E. J., Refinement of macromolecular structures by the maximum-likelihood method. Acta Crystallogr D Biol Crystallogr 53 (Pt 3), 240 (1997).

B. Nagar, W. G. Bornmann, P. Pellicena, T. Schindler, D. R. Veach, W. T. Miller, B. Clarkson, J. Kuriyan, Cancer Res 2002, 62, 4236.

Nienaber, V. L. et al., Discovering novel ligands for macromolecules using X-ray crystallographic screening. Nature biotechnology 18 (10), 1105 (2000).

Pargellis, C., Tong, L., Churchill, L., Cirillo, P. F., Gilmore, T., Graham, A. G., Grob, P. M., Hickey, E. R., Moss, N., Pav, S., and Regan, J. (2002) Inhibition of p38 MAP kinase by utilizing a novel allosteric binding site, Nature structural biology 9, 268-272.

W. A Pearson, Rapid and Sensitive Sequence Comparison with FASTP and FASTA, in Methods in Enzymology, ed. R. Doolittle Academic Press, San Diego) 183(1990)63-98.

Pierce AC, ter Haar E, Binch HM, Kay DP, Patel SR, Li P. J Med Chem. 2005 Feb 24;48(4):1278-81.

Read, R. J., Pushing the boundaries of molecular replacement with maximum likelihood. Acta Crystallogr D 57 (Pt 10), 1373 (2001).

Reich, M. et al., GenePattern 2.0. Nat Genet 38 (5), 500 (2006).

Regan, J., Breitfelder, S., Cirillo, P., Gilmore, T., Graham, A. G., Hickey, E., Klaus, B., Madwed, J., Moriak, M., Moss, N., Pargellis, C., Pav, S., Proto, A., Swinamer, A., Tong, L., and Torcellini, C. (2002) Pyrazole urea-based inhibitors of p38 MAP kinase: from lead compound to clinical candidate, Journal of medicinal chemistry 45, 2994-3008.

Regan, J., Pargellis, C. A., Cirillo, P. F., Gilmore, T., Hickey, E. R., Peet, G. W., Proto, A., Swinamer, A., and Moss, N. (2003) The kinetics of binding to p38MAP kinase by analogues of BIRB 796, Bioorganic & medicinal chemistry letters 13, 3101-3104.

Rendell, D. (1987). Fluorescence and Phosphorescence.

Crown Richieri, G. V., Ogata, R. T., and Kleinfeld, A. M. (1999) The measurement of free fatty acid concentration with the fluorescent probe ADIFAB: a practical guide for the use of the ADIFAB probe, Molecular and cellular biochemistry 192, 87-94.M.

Saraste, P. R. Sibbald, A. Wittinghofer, Trends Biochem Sci 1990, 15, 430. Schuttelkopf, A. W. and van Aalten, D. M., PRODRG: a tool for high-throughput crystallography of protein-ligand complexes. Acta Crystallogr D Biol Crystallogr 60 (Pt 8), 1355 (2004).

Sharma, A. and Schulman, S. G. (1999). Introduction to Fluorescence Spectroscopy. Wiley interscience.Sullivan, J. E., Holdgate, G. A., Campbell, D., Timms, D., Gerhardt, S., Breed, J., Breeze, A. L., Bermingham, A., Pauptit, R. A., Norman, R. A., Embrey, K. J., Read, J., VanScyoc, W. S., and Ward, W. H. (2005) Prevention of MKK6-dependent activation by binding to p38alpha MAP kinase, Biochemistry 44, 16475-16490.

M. A. Seeliger, B. Nagar, F. Frank, X. Cao, M. N. Henderson, J. Kuriyan, Structure 2007, 15, 299.Shuker, S. B., Hajduk, P. J., Meadows, R. P., and Fesik, S. W., Discovering high-affinity ligands for proteins: SAR by NMR. Science 274 (5292), 1531 (1996).

J. R. Simard, S. Klüter, C. Grütter, M. Getlik, M. Rabiller, H. B. Rode, D. Rauh, Nat Chem Biol 2009, in press.

Tamayo, N, Liao, L., Goldberg,M., Powers, D., Tudor, Y-Y, Yu, V., Wong, L. M., Henkle, B., Middleton, S., Syed, R., Harvey, T., Jang, G., Hungate, R. and Celia Dominguez (2005). Design and synthesis of potent pyridazine inhibitors of p38 MAP kinase, Bioorganic & Medicinal Chemistry Letters 15:2409-2413.

Taylor, S. S. and Radzio-Andzelm, E. (1994) Three protein kinase structures define a common motif. Structure 2, 345-355; 43.Vogtherr, M., Saxena, K., Hoelder, S., Grimme, S., Betz, M., Schieborr, U., Pescatore, B., Robin, M., Delarbre, L., Langer, T., Wendt, K. U., and Schwalbe, H. (2006) NMR characterization of kinase p38 dynamics in free and ligand-bound forms, Angewandte Chemie (International ed 45, 993-997.Yeatman, T. J., A renaissance for SRC. Nat Rev Cancer 4 (6), 470 (2004).

Tokarski, J.S., Newitt, J., Chang, C.Y.J., Cheng, J.D., Wittekind, M., Kiefer, S.E., Kish, K., Lee, F.Y.F., Borzilerri, R., Lombardo, L.J., Xie, D., Zhang, Y., Klei, H.E. (2006) CANCER RES. 66: 5790-5797.

Wan, P. T., Garnett, M. J., Roe, S. M., Lee, S., Niculescu-Duvaz, D., Good, V. M., Jones, C. M., Marshall, C. J., Springer, C. J., Barford, D., Marais, R., Cell 2004, 116, 855.Wong, L., Lieser, S. A., Miyashita, O., Miller, M., Tasken, K., Onuchic, J. N., Adams, J. A., V. L. Woods, Jr., P. A. Jennings, J Mol Biol 2005, 351, 131.

Yem, A. W., Epps, D. E., Mathews, W. R., Guido, D. M., Richard, K. A., Staite, N. D., and Deibel, M. R., Jr. (1992) Site-specific chemical modification of interieukin-1 beta by acrylodan at cysteine 8 and lysine 103, The Journal of biological chemistry 267, 3122-3128.

J. Zhang, P. L. Yang, N. S. Gray, Nat. Rev. Cancer 2009, 9, 28.

## Claims

1. A kinase labeled at an amino acid naturally present or introduced in the P-loop of said kinase, wherein said labeling is effected at a free thiol or amino group of said amino acid and said label is

   (a) a thiol- or amino-reactive fluorophore sensitive to polarity changes in its environment; or
   (b) a thiol-reactive spin label, an isotope or an isotope-enriched thiol- or amino-reactive label;
   such that said fluorophore, spin label, isotope or isotope-enriched label does not inhibit the catalytic activity and does not interfere with the stability of the kinase.

2. The kinase of claim 1, which is a serine/threonine or tyrosine kinase.

3. The kinase of claim 1 or 2, which is MEK kinase, CSK, an Aurora kinase, GSK-3beta, cSrc, EGFR, Abl, DDR1, AKT, LCK, a CDK, p38$\alpha$ or another MAPK.

4. The kinase of any one of claims 1 to 3, wherein the amino acid labeled is cysteine, lysine, arginine or histidine.

5. The kinase of any one of claims 1 to 4, wherein one or more solvent-exposed cysteines present outside the P-loop are deleted or replaced.

6. The kinase of any one of claims 3 to 5, which is p38α and wherein a cysteine to be labeled is introduced at position 35 of SEQ ID NO: 1 and preferably wherein the cysteines at position 119 and 162 of SEQ ID NO: 1 are replaced with another amino acid.

7. The kinase of any one of claims 1 to 6, wherein the thiol- or amino- reactive fluorophore is a di-substituted naphthalene compound, a coumarin-based compound, a benzoxadiazole-based compound, a dapoxyl-based compound, a biocytin-based compound, a fluorescein, a sulfonated rhodamine-based compound, Atto fluorophores or Lucifer Yellow or derivatives thereof which exhibit a sensitivity to environmental changes.

8. The kinase of any one of claims 1 to 6, wherein the thiol-reactive spin-label is a nitroxide radical.

9. A method of screening for kinase inhibitors comprising

   (a) providing a kinase according to any one of claims 1 to 8
   (b) contacting said fluorescently or spin-labeled or isotope-labeled kinase with a candidate inhibitor;
   (c) recording the fluorescence emission signal at one or more wavelengths or a spectrum of said fluorescently labeled kinase of step (a) and step (b) upon excitation: or
   (c)' recording the electron paramagnetic resonance (EPR) or nuclear magnetic resonance (NMR) spectra of said spin-labeled or isotope-labeled kinase of step (a) and step (b); and
   (d) comparing the fluorescence emission signal at one or more wavelengths or the spectra recorded in step (c) or the EPR or NMR spectra recorded in step (c)';
   wherein a difference in the fluorescence intensity at at least one wavelength, preferably at the emission maximum, and/or a shift in the fluorescence emission wavelength in the spectra of said fluorescently labeled kinase obtained in step (c), or an alteration in the EPR or NMR spectra of said spin-labeled or isotope-labeled kinase obtained in step (c)' indicates that the candidate inhibitor is a kinase inhibitor.

10. A method of determining the kinetics of ligand binding and/or of association or dissociation of a kinase inhibitor comprising

   (a) contacting a fluorescently labeled kinase according to any one of claims 1 to 8 with different concentrations of an inhibitor; or
   (a)' contacting a fluorescently labeled kinase according to any one of claims 1 to 8 bound to an inhibitor with different concentrations of unlabeled kinase;
   (b) recording the fluorescence emission signal at one or more wavelengths or a spectrum of said fluorescently labeled kinase for each concentration upon excitation;
   (c) determining the rate constant for each concentration from the fluorescence emission signals at one or more wavelengths or the spectra recorded in step (b); or
   (c1) determining the $K_d$ from the fluorescence emission signal at one
   or more wavelengths or the spectra recorded in step (b) for each concentration of inhibitor; or
   (c2) determining the $K_a$ from the fluorescence emission signal at one
   or more wavelengths or the spectra recorded in step (b) for each concentration of unlabelled kinase;
   (d) directly determining the $k_{on}$ and/or extrapolating the $k_{off}$ from the rate constants determined in step (c) from the signals or spectra for the different concentrations of inhibitor obtained in step (b); or (d)' directly determining the $k_{off}$ and /or extrapolating the $k_{on}$ from the rate constants determined in step (c) from the signals or spectra for the different concentrations of unlabeled kinase obtained in step (b); and
   (e) optionally calculating the $K_d$ and/or Ka from $k_{on}$ and $k_{off}$ obtained in step (d) or (d)'.

11. A method of determining the dissociation or association of a kinase inhibitor comprising

   (a) contacting a spin-labeled or isotope-labeled kinase according to any one of claims 1 to 8 with different concentrations of an inhibitor; or
   (a)' contacting a spin-labeled or isotope-labeled kinase according to any one of claims 1 to 8 bound to an inhibitor with different concentrations of unlabelled kinase;
   (b) recording the EPR or NMR spectrum of said spin-labeled or isotope-labeled kinase for each concentration of inhibitor and/or unlabelled kinase; and
   (c) determining the $K_d$ from the EPR or NMR spectra recorded in step (b) for the different concentrations of inhibitor; or

(c)' determining the $K_a$ from the EPR or NMR spectra recorded in step (b) for the different concentrations of unlabeled kinase.

**12.** A method of generating a mutated kinase suitable for the screening of kinase inhibitors comprising

(a) replacing solvent exposed amino acids having a free thiol or amino group, if any, present in a kinase of interest outside the P-loop and/or amino acids having a free thiol or amino group at an unsuitable position within the P-loop with an amino acid not having a free thiol or amino group;

(b) mutating an amino acid in the P-loop of said kinase of interest to an amino acid having a free thiol or amino group if no amino acid having a free thiol or amino group is present in the P-loop ;

(c) labeling the kinase of interest with a thiol- or amino- reactive fluorophore sensitive to polarity changes in its environment, a thiol-reactive spin label, an isotope or an isotope-enriched thiol- or amino-reactive label such that said fluorophore, spin label, isotope or isotope-enriched label does not inhibit the catalytic activity of the kinase and/or does not interfere with the stability of the kinase;

(d) contacting the kinase obtained in step (c) with a known inhibitor of said kinase; and

(e) recording the fluorescence emission signal at one or more wavelengths or a spectrum of said fluorescently labeled kinase of step (c) and (d) upon excitation or

(e)' recording the EPR or NMR spectra of said spin-labeled kinase of step (c) and (d);

(f) comparing the fluorescence emission spectra recorded in step (e)

or the EPR or NMR spectra recorded in step (e)';

wherein a difference in the fluorescence intensity at at least one wavelength, preferably at the emission maximum, and/or a shift in the fluorescence emission wavelength in the spectra of said fluorescently labeled kinase obtained in step (e), or an alteration in the EPR or NMR spectra of said spin-labeled or isotope-labeled kinase obtained in step (e)' indicates that the kinase is suitable for the screening for kinase inhibitors.

**13.** The method of any one of claims 9 to 12, wherein the kinase inhibitor binds either partially or fully to the allosteric site adjacent to the ATP binding site of the kinase.

**14.** A method for identifying a kinase inhibitor binding either partially or fully to the allosteric site adjacent to the ATP binding site of a kinase comprising

(a) screening for an inhibitor according to the method of claim 10, and

(b) determining the rate constant of an inhibitor identified in step (a), wherein a rate constant of <0.140 $s^{-1}$ determined in step (b) indicates that the kinase inhibitor identified binds either partially or fully to the allosteric site adjacent to the ATP binding site of the kinase.

**15.** The kinase of any one of claims 1 to 8 or the method of any one of claims 9 to 14, wherein the kinase is labeled at a cysteine naturally present or introduced in the P-loop.

**16.** The method of any one of claims 9 or 12 to 15, further comprising optimizing the pharmacological properties of a compound identified as inhibitor of said kinase.

**17.** The method of claim 16, wherein the optimization comprises modifying an inhibitor identified as inhibitor of said kinase to achieve:

a) modified spectrum of activity, organ specificity, and/or

b) improved potency, and/or

c) decreased toxicity (improved therapeutic index), and/or

d) decreased side effects, and/or

e) modified onset of therapeutic action, duration of effect, and/or

f) modified pharmacokinetic parameters (absorption, distribution, metabolism and excretion), and/or

g) modified physico-chemical parameters (solubility, hygroscopicity, color, taste, odor, stability, state), and/or

h) improved general specificity, organ/tissue specificity, and/or

i) optimized application form and route

by

a. esterification of carboxyl groups, or

b. esterification of hydroxyl groups with carboxylic acids, or

c. esterification of hydroxyl groups to, e.g. phosphates, pyrophosphates or sulfates or hemi-succinates, or

d. formation of pharmaceutically acceptable salts, or

e. formation of pharmaceutically acceptable complexes, or

f. synthesis of pharmacologically active polymers, or

g. introduction of hydrophilic moieties, or

h. introduction/exchange of substituents on aromates or side chains, change of substituent pattern, or

i. modification by introduction of isosteric or bioisosteric moieties, or

j. synthesis of homologous compounds, or

k. introduction of branched side chains, or

l. conversion of alkyl substituents to cyclic analogues, or

m. derivatization of hydroxyl groups to ketales, acetales, or

n. N-acetylation to amides, phenylcarbamates, or

o. synthesis of Mannich bases, imines, or

p. transformation of ketones or aldehydes to Schiffs bases, oximes,

acetales, ketales, enolesters, oxazolidines, thiazolidines or combinations thereof.

Figure 1 a) b) c)

Figure 2

Figure 3

**Figure 4**

Type I: SB203580

Type II: BIRB-796

RL40

Type I: dasatinib

Type III: RL36

Scios-469

EP 2 241 619 A1

| Compound | Inhibitor Type | $\Delta I_{std}$* | $\Delta R_{max}$** | 100 nM single dose | | Kd (nM) |
| | | | | Binding $t_{1/2}$ (sec) | Dissociation $t_{1/2}$ (sec) | |
|---|---|---|---|---|---|---|
| dasatinib | I | 0.030 ±0.015 | 0.090 ±0.038 | ND | ND | ND |
| SB203580 | I | **0.712 ±0.048** | 0.232 ±0.011 | < 2 | < 10 | 0.0168 ±0.0042 |
| RL40 | I | **1.572 ±0.325** | 0.564 ±0.037 | < 2 | < 10 | 1.032 ±0.181 |
| Scios-469 | I | **1.117 ±0.020** | 0.254 ±0.014 | < 2 | < 10 | 0.0082 ±0.0029 |
| BIRB-796 | II | **0.550±0.095** | 0.301±0.083 | 153.4 | 2440.7 | 0.0095±0.0027 |
| RL36 | III | **0.621 ±0.076** | 0.367 ±0.034 | 147.5 | 1575.3 | 0.0152±0.0008 |

# Figure 5

a)

b)

c)

**EUROPEAN SEARCH REPORT**

Application Number

EP 09 00 5492

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LANGER THOMAS ET AL: "NMR backbone assignment of a protein kinase catalytic domain by a combination of several approaches: application to the catalytic subunit of cAMP-dependent protein kinase" CHEMBIOCHEM - A EUROPEAN JOURNAL OF CHEMICAL BIOLOGY, WILEY VCH, WEINHEIM, DE, vol. 5, no. 11, 5 November 2004 (2004-11-05), pages 1508-1516, XP002503169 ISSN: 1439-4227 * abstract * ----- | 1-4,9, 11,13 | INV. C12N9/16 C12Q1/48 |
| X | JOSEPH R. LAKOWICZ: "Principles of fluorescence spectroscopy" 2006, SPRINGER , XP000918225 ISBN: 0387312781 see section 13.5.3 wherein alexa 546 labeled kinase is mentionned * page 459 * ----- | 1-4 | |
| X | LEVINE H III ET AL: "A MONOCLONAL ANTIBODY AGAINST BRAIN CALMODULIN-DEPENDENT PROTEIN KINASE TYPE II DETECTS PUTATIVE CONFORMATION CHANGES INDUCED BY CALCIUM CALMODULIN" BIOCHEMISTRY, vol. 27, no. 17, 1988, pages 6612-6617, XP002540825 ISSN: 0006-2960 * abstract * ----- -/-- | 1-4 | TECHNICAL FIELDS SEARCHED (IPC) C12N C12Q |

The present search report has been drawn up for all claims

| Place of search | Date of completion of the search | Examiner |
|---|---|---|
| Munich | 11 August 2009 | Hinchliffe, Philippe |

CATEGORY OF CITED DOCUMENTS

X : particularly relevant if taken alone
Y : particularly relevant if combined with another
　　document of the same category
A : technological background
O : non-written disclosure
P : intermediate document

T : theory or principle underlying the invention
E : earlier patent document, but published on, or
　　after the filing date
D : document cited in the application
L : document cited for other reasons
.................................................................
& : member of the same patent family, corresponding
　　document

EPO FORM 1503 03.82 (P04C01)

**Europäisches Patentamt**
**European Patent Office**
**Office européen des brevets**

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FIRST E A ET AL: "SELECTIVE MODIFICATION OF THE CATALYTIC SUBUNIT OF CAMP-DEPENDENT PROTEIN KINASE WITH SULFHYDRYL-SPECIFIC FLUORESCENT PROBES" BIOCHEMISTRY, AMERICAN CHEMICAL SOCIETY, EASTON, PA.; US, vol. 28, no. 8, 1 January 1989 (1989-01-01), pages 3598-3605, XP002073139 ISSN: 0006-2960 * abstract * | 1-4 | |
| A | KAPAHI PANKAJ ET AL: "Inhibition of NF-kappaB activation by arsenite through reaction with a critical cysteine in the activation loop of IkappaB kinase" JOURNAL OF BIOLOGICAL CHEMISTRY, AMERICAN SOCIETY OF BIOLOCHEMICAL BIOLOGISTS, BIRMINGHAM, US, vol. 275, no. 46, 1 November 2000 (2000-11-01), pages 36062-36066, XP002503167 ISSN: 0021-9258 [retrieved on 2000-08-30] * page 36065, column 1, paragraph 2; figure 4 * | 1-17 | |

TECHNICAL FIELDS SEARCHED (IPC)

The present search report has been drawn up for all claims

| Place of search | Date of completion of the search | Examiner |
|---|---|---|
| Munich | 11 August 2009 | Hinchliffe, Philippe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 2 241 619 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 08013340 A

- EP 08020341 A

### Non-patent literature cited in the description

- **Kubinyi.** Hausch-Analysis and Related Approaches. VCH Verlag, 1992
- **Holzgrabe ; Bechtold.** *Deutsche Apotheker Zeitung,* 2000, vol. 140 (8), 813-823
- **Annis, D. A. ; Nazef, N. ; Chuang, C. C. ; Scott, M. P. ; Nash, H. M.** A general technique to rank protein-ligand binding affinities and determine allosteric versus direct binding site competition in compound mixtures. *Journal of the American Chemical Society,* 2004, vol. 126, 15495-15503
- **R. T. Aimes ; W. Hemmer ; S. S. Taylor.** *Biochemistry,* 2000, vol. 39, 8325
- **B. Apsel ; J. A. Blair ; B. Gonzalez ; T. M. Nazif ; M. E. Feldman ; B. Aizenstein ; R. Hoffman ; R. L. Williams ; K. M. Shokat ; Z. A. Knight.** *Nat Chem Biol,* 2008, vol. 4, 691
- **A. C. Backes ; B. Zech ; B. Felber ; B. Klebl ; G. Müller.** *Expert Opin Drug Discovery,* 2008, vol. 3, 1409
- **A. C. Backes ; B. Zech ; B. Felber ; B. Klebl ; G. Müller.** *Expert Opin Drug Discovery,* 2008, vol. 3, 1427
- **J. A. Blair ; D. Rauh ; C. Kung ; C. H. Yun ; Q. W. Fan ; H. Rode ; C. Zhang ; M. J. Eck ; W. A. Weiss ; K. M. Shokat.** *Nat Chem Biol,* 2007, vol. 3, 229
- **R. A. Copeland ; D. L. Pompliano ; T. D. Meek.** *Nat Rev Drug Discov,* 2006, vol. 5, 730
- **Dar, A. C. ; Lopez, M. S. ; Shokat, K. M.** Small Molecule Recognition of c-Src via the Imatinib-Binding Conformation. *Chem Biol,* 2008, vol. 15 (10), 1015
- **DeLano, W.L.** The PyMOL Molecular Graphics System. *http:llwww.pymol.org,* 2002
- **de Lorimier, R. M. ; Smith, J. J. ; Dwyer, M. A. ; Looger, L. L. ; Sali, K. M. ; Paavola, C. D. ; Rizk, S. S. ; Sadigov, S. ; Conrad, D. W. ; Loew, L.** Construction of a fluorescent biosensor family. *Protein Sci,* 2002, vol. 11, 2655-2675

- **Dumas, J. ; Hatoum-Mokdad, H. ; Sibley, R. ; Riedl, B. ; Scott, W. J. ; Monahan, M. K. ; Lowinger, T. B. ; Brennan, C. ; Natero, R. ; Turner, T.** 1-Phenyl-5-pyrazolyl ureas: potent and selective p38 kinase inhibitors. *Bioorganic & medicinal chemistry letters,* 2000, vol. 10, 2051-2054
- **Emsley, P. ; Cowtan, K.** Coot: model-building tools for molecular graphics. *Acta Crystallogr D Biol Crystallogr,* 2004, vol. 60, 2126
- **M. J. Garnett ; R. Marais.** *Cancer Cell,* 2004, vol. 6, 313
- **Gauglitz, G. ; Vo-Dinh, T.** Handbook of spectroscopy. Wiley-VCH, 2003
- **Gschwind, A. ; Fischer, O. M. ; Ullrich, A.** The discovery of receptor tyrosine kinases: targets for cancer therapy. *Nat Rev Cancer,* 2004, vol. 4 (5), 361
- **S. K. Hanks ; T. Hunter.** *FASEB J,* 1995, vol. 9, 576
- **Hibbs, R. E. ; Talley, T. T. ; Taylor, P.** Acrylodan-conjugated cysteine side chains reveal conformational state and ligand site locations of the acetylcholine-binding protein. *The Journal of biological chemistry,* 2004, vol. 279, 28483-28491
- **Johnson, L. N. ; Noble, M. E. M. ; Owen, D. J.** Active and inactive protein kinases : structural basis for regulation. *Cell,* 1996, vol. 85, 149-158
- **Johnson, L. N. ; Lewis, R. E.** Structural basis for control by phosphorylation. *Chem Rev.,* August 2001, vol. 101 (8), 2209-42
- **Kabsch, W.** Automatic processing of rotation diffraction data from crystals of initially unknown symmetry and cell constants. *J. Appl. Cryst.,* 1993, vol. 26, 795
- **Lakowicz, J. R.** Principles of Fluorescence Spectroscopy. Kluwer Academic / Plenum Publishers, 1999
- **Laskowski, R. A. ; MacArthur, M. W. ; Moss, D. S. ; Thornton, J. M.** PROCHECK: a program to check the stereochemical quality of protein structures. *J Appl Cryst,* 1993, vol. 26, 283
- **Larkin, M. A. et al.** Clustal W and Clustal X version 2.0. *Bioinformatics,* 2007, vol. 23 (21), 2947

**34**

- **Levinson, N. M. et al.** A Src-like inactive conformation in the abl tyrosine kinase domain. *PLoS Biol,* 2006, vol. 4 (5), e144
- **Liu, Y. ; Gray, N. S.** Rational design of inhibitors that bind to inactive kinase conformations. *Nat Chem Biol,* 2006, vol. 2 (7), 358
- **M. Mapelli ; L. Massimiliano ; C. Crovace ; M. A. Seeliger ; L. H. Tsai ; L. Meijer ; A. Musacchio.** *J Med Chem,* 2005, vol. 48, 671
- **Michalczyk, A. et al.** Structural insights into how irreversible inhibitors can overcome drug resistance in EGFR. *Bioorg Med Chem,* 2008, vol. 16 (7), 3482
- **Murshudov, G. N. ; Vagin, A. A. ; Dodson, E. J.** Refinement of macromolecular structures by the maximum-likelihood method. *Acta Crystallogr D Biol Crystallogr,* 1997, vol. 53, 240
- **B. Nagar ; W. G. Bornmann ; P. Pellicena ; T. Schindler ; D. R. Veach ; W. T. Miller ; B. Clarkson ; J. Kuriyan.** *Cancer Res,* 2002, vol. 62, 4236
- **Nienaber, V. L. et al.** Discovering novel ligands for macromolecules using X-ray crystallographic screening. *Nature biotechnology,* 2000, vol. 18 (10), 1105
- **Pargellis, C. ; Tong, L. ; Churchill, L. ; Cirillo, P. F. ; Gilmore, T. ; Graham, A. G. ; Grob, P. M. ; Hickey, E. R. ; Moss, N. ; Pav, S.** Inhibition of p38 MAP kinase by utilizing a novel allosteric binding site. *Nature structural biology,* 2002, vol. 9, 268-272
- Rapid and Sensitive Sequence Comparison with FASTP and FASTA. **W. A Pearson.** Methods in Enzymology. Academic Press, 1990, vol. 183, 63-98
- **Pierce AC ; ter Haar E ; Binch HM ; Kay DP ; Patel SR ; Li P.** *J Med Chem.,* 24 February 2005, vol. 48 (4), 1278-81
- **Read, R. J.** Pushing the boundaries of molecular replacement with maximum likelihood. *Acta Crystallogr D,* 2001, vol. 57, 1373
- **Reich, M. et al.** GenePattern 2.0. *Nat Genet,* 2006, vol. 38 (5), 500
- **Regan, J. ; Breitfelder, S. ; Cirillo, P. ; Gilmore, T. ; Graham, A. G. ; Hickey, E. ; Klaus, B. ; Madwed, J. ; Moriak, M. ; Moss, N.** Pyrazole urea-based inhibitors of p38 MAP kinase: from lead compound to clinical candidate. *Journal of medicinal chemistry,* 2002, vol. 45, 2994-3008
- **Regan, J. ; Pargellis, C. A. ; Cirillo, P. F. ; Gilmore, T. ; Hickey, E. R. ; Peet, G. W. ; Proto, A. ; Swinamer, A. ; Moss, N.** The kinetics of binding to p38MAP kinase by analogues of BIRB 796. *Bioorganic & medicinal chemistry letters,* 2003, vol. 13, 3101-3104
- **Rendell, D.** *Fluorescence and Phosphorescence,* 1987
- **Crown Richieri, G. V. ; Ogata, R. T. ; Kleinfeld, A. M.** The measurement of free fatty acid concentration with the fluorescent probe ADIFAB: a practical guide for the use of the ADIFAB probe. *Molecular and cellular biochemistry,* 1999, vol. 192, 87-94.M
- **Saraste, P. ; R. Sibbald ; A. Wittinghofer.** *Trends Biochem Sci,* 1990, vol. 15, 430
- **Schuttelkopf, A. W. ; van Aalten, D. M.** PRODRG: a tool for high-throughput crystallography of protein-ligand complexes. *Acta Crystallogr D Biol Crystallogr,* 2004, vol. 60, 1355
- **Sharma, A. ; Schulman, S. G.** Introduction to Fluorescence Spectroscopy. Wiley interscience, 1999
- **Sullivan, J. E. ; Holdgate, G. A. ; Campbell, D. ; Timms, D. ; Gerhardt, S. ; Breed, J. ; Breeze, A. L. ; Bermingham, A. ; Pauptit, R. A. ; Norman, R. A.** Prevention of MKK6-dependent activation by binding to p38alpha MAP kinase. *Biochemistry,* 2005, vol. 44, 16475-16490
- **M. A. Seeliger ; B. Nagar ; F. Frank ; X. Cao ; M. N. Henderson ; J. Kuriyan.** *Structure,* 2007, vol. 15, 299
- **Shuker, S. B. ; Hajduk, P. J. ; Meadows, R. P. ; Fesik, S. W.** Discovering high-affinity ligands for proteins: SAR by NMR. *Science,* 1996, vol. 274 (5292), 1531
- **J. R. Simard ; S. Klüter ; C. Grütter ; M. Getlik ; M. Rabiller ; H. B. Rode ; D. Rauh.** *Nat Chem Biol,* 2009
- **Tamayo, N ; Liao, L. ; Goldberg,M. ; Powers, D. ; Tudor, Y-Y ; Yu, V. ; Wong, L. M. ; Henkle, B. ; Middleton, S. ; Syed, R.** Design and synthesis of potent pyridazine inhibitors of p38 MAP kinase. *Bioorganic & Medicinal Chemistry Letters,* 2005, vol. 15, 2409-2413
- **Taylor, S. S. ; Radzio-Andzelm, E.** Three protein kinase structures define a common motif. *Structure,* 1994, vol. 2, 345-355
- NMR characterization of kinase p38 dynamics in free and ligand-bound forms. **Vogtherr, M. ; Saxena, K. ; Hoelder, S. ; Grimme, S. ; Betz, M. ; Schieborr, U. ; Pescatore, B. ; Robin, M. ; Delarbre, L. ; Langer, T.** Angewandte Chemie. 2006, 993-997
- **Yeatman, T. J.** A renaissance for SRC. *Nat Rev Cancer,* 2004, vol. 4 (6), 470
- **Tokarski, J.S. ; Newitt, J. ; Chang, C.Y.J. ; Cheng, J.D. ; Wittekind, M. ; Kiefer, S.E. ; Kish, K. ; Lee, F.Y.F. ; Borzilerri, R. ; Lombardo, L.J.** *CANCER RES.,* 2006, vol. 66, 5790-5797
- **Wan, P. T. ; Garnett, M. J. ; Roe, S. M. ; Lee, S. ; Niculescu-Duvaz, D. ; Good, V. M. ; Jones, C. M. ; Marshall, C. J. ; Springer, C. J. ; Barford, D.** *Cell,* 2004, vol. 116, 855
- **Wong, L. ; Lieser, S. A. ; Miyashita, O. ; Miller, M. ; Tasken, K. ; Onuchic, J. N. ; Adams, J. A. ; V. L. Woods, Jr. ; P. A. Jennings.** *J Mol Biol,* 2005, vol. 351, 131

- **Yem, A. W. ; Epps, D. E. ; Mathews, W. R. ; Guido, D. M. ; Richard, K. A. ; Staite, N. D. ; Deibel, M. R., Jr.** Site-specific chemical modification of interieukin-1 beta by acrylodan at cysteine 8 and lysine 103. *The Journal of biological chemistry,* 1992, vol. 267, 3122-3128

- **J. Zhang ; P. L. Yang ; N. S. Gray.** *Nat. Rev. Cancer,* 2009, vol. 9, 28

SEQUENCE LISTING

<110> Max-Planck-Gesellschaft zur Förderung der Wissenschaften e.V.

<120> Development of fluorescently P-loop labeled kinases for screening of inhibitors

<130> R1171 EP

<160> 1

<170> PatentIn version 3.3

<210> 1
<211> 360
<212> PRT
<213> Homo sapiens

<400> 1

Met Ser Gln Glu Arg Pro Thr Phe Tyr Arg Gln Glu Leu Asn Lys Thr
1               5                   10                  15

Ile Trp Glu Val Pro Glu Arg Tyr Gln Asn Leu Ser Pro Val Gly Ser
            20                  25                  30

Gly Ala Tyr Gly Ser Val Cys Ala Ala Phe Asp Thr Lys Thr Gly Leu
        35                  40                  45

Arg Val Ala Val Lys Lys Leu Ser Arg Pro Phe Gln Ser Ile Ile His
    50                  55                  60

Ala Lys Arg Thr Tyr Arg Glu Leu Arg Leu Leu Lys His Met Lys His
65                  70                  75                  80

Glu Asn Val Ile Gly Leu Leu Asp Val Phe Thr Pro Ala Arg Ser Leu
                85                  90                  95

Glu Glu Phe Asn Asp Val Tyr Leu Val Thr His Leu Met Gly Ala Asp
            100                 105                 110

Leu Asn Asn Ile Val Lys Cys Gln Lys Leu Thr Asp Asp His Val Gln
            115                 120                 125

Phe Leu Ile Tyr Gln Ile Leu Arg Gly Leu Lys Tyr Ile His Ser Ala
    130                 135                 140

Asp Ile Ile His Arg Asp Leu Lys Pro Ser Asn Leu Ala Val Asn Glu
145                 150                 155                 160

Asp Cys Glu Leu Lys Ile Leu Asp Phe Gly Leu Ala Arg His Thr Asp
                165                 170                 175

Asp Glu Met Thr Gly Tyr Val Ala Thr Arg Trp Tyr Arg Ala Pro Glu
            180                 185                 190

```
Ile Met Leu Asn Trp Met His Tyr Asn Gln Thr Val Asp Ile Trp Ser
        195             200             205

Val Gly Cys Ile Met Ala Glu Leu Leu Thr Gly Arg Thr Leu Phe Pro
    210             215             220

Gly Thr Asp His Ile Asp Gln Leu Lys Leu Ile Leu Arg Leu Val Gly
225             230             235             240

Thr Pro Gly Ala Glu Leu Leu Lys Lys Ile Ser Ser Glu Ser Ala Arg
            245             250             255

Asn Tyr Ile Gln Ser Leu Thr Gln Met Pro Lys Met Asn Phe Ala Asn
            260             265             270

Val Phe Ile Gly Ala Asn Pro Leu Ala Val Asp Leu Leu Glu Lys Met
    275             280             285

Leu Val Leu Asp Ser Asp Lys Arg Ile Thr Ala Ala Gln Ala Leu Ala
    290             295             300

His Ala Tyr Phe Ala Gln Tyr His Asp Pro Asp Asp Glu Pro Val Ala
305             310             315             320

Asp Pro Tyr Asp Gln Ser Phe Glu Ser Arg Asp Leu Leu Ile Asp Glu
            325             330             335

Trp Lys Ser Leu Thr Tyr Asp Glu Val Ile Ser Phe Val Pro Pro Pro
            340             345             350

Leu Asp Gln Glu Glu Met Glu Ser
        355             360
```